# EUROPÄISCHE PATENTSCHRIFT

(11) **EP 2 558 084 B1**
(45) Veröffentlichungstag und Bekanntmachung des Hinweises auf die Patenterteilung: **06.03.2019**
(21) Anmeldenummer: 11717483.9
(22) Anmeldetag: 14.04.2011
(51) Int. Cl.: A61K 31/122, A61K 31/455, A61P 9/00, A61P 9/10, A23L 33/10, A23L 33/15

(54) **KOMBINATION AUS VITAMIN K UND NIKOTINAMID FÜR DIE VERWENDUNG BEI ERKRANKUNGEN, DIE MIT EXTRAOSSÄRER KALZIFIZIERUNG EINHERGEHEN**
COMBINATION OF VITAMIN K AND NICOTINAMIDE FOR THE USE IN DISEASES WHICH IMPLY EXTRAOSSEOUS CALCIFICATION
COMBINAISON DU VITAMIN K ET DE L'AMIDE NICOTINIQUE POUR L'UTILISATION DANS DES MALADIES QUI IMPLIQUENT UNE CALCIFICATION EXTRA-OSSEUSE

(30) Priorität: 19.04.2010 US 325507 P; 15.04.2010 DE 102010015242
(43) Veröffentlichungstag der Anmeldung: 20.02.2013
(73) Patentinhaber: Fresenius Medical Care Deutschland GmbH, 61352 Bad Homburg (DE)
(72) Erfinder: PASSLICK-DEETJEN, Jutta, 35392 Giessen (DE); SCHULZE, Friedrich, 61267 Neu-Anspach (DE)
(74) Vertreter: Bülle, Jan
(86) Internationale Anmeldenummer: PCT/EP2011/001883
(87) Internationale Veröffentlichungsnummer: WO 2011/141113

(56) Entgegenhaltungen:
- WO-A1-2011/013138
- DE-A1-102007 003 524
- KR-A- 20080 008 155
- US-A1- 2008 317 725
- WALLIN R ET AL: "Effects of the blood coagulation vitamin K as an inhibitor of arterial calcification", THROMBOSIS RESEARCH, TARRYTOWN, NY, US, Bd. 122, Nr. 3, 1. Januar 2008 (2008-01-01), Seiten 411-417, XP022797478, ISSN: 0049-3848, DOI: 10.1016/J.THROMRES.2007.12.005 [gefunden am 2008-01-30]
- SAITO EIJI ET AL: "Treatment with vitamin k(2) combined with bisphosphonates synergistically inhibits calcification in cultured smooth muscle cells", JOURNAL OF ATHEROSCLEROSIS AND THROMBOSIS, 1. Dezember 2007 (2007-12-01), XP002657394,
- SCHURGERS LEON J ET AL: "Matrix Gla-protein: The calcification inhibitor in need of vitamin K", THROMBOSIS AND HAEMOSTATIS, 1. Oktober 2008 (2008-10-01), XP002657395, in der Anmeldung erwähnt
- KRUEGER THILO ET AL: "Vitamin K deficiency in CKD patients: a modifiable risk factor for vascular calcification?", KIDNEY INTERNATIONAL, 1. Juli 2009 (2009-07-01), XP002657396,

## Beschreibung

Die Erfindung betrifft die Prävention oder Behandlung einer extraossären Kalzifizierung, welche im Zusammenhang mit verschiedenen Erkrankungen auftreten kann, beziehungsweise die Verhinderung von Folgeerscheinungen einer extraossären Kalzifizierung, durch Verabreichung einer Vitamin-K-Komponente und einer Nikotinamid-Komponente. Ferner betrifft die Erfindung eine pharmazeutische Zusammensetzung und eine pharmazeutische Darreichungsform, welche eine Kombination aus einer Vitamin-K-Komponente und einer Nikotinamid-Komponente enthalten, bevorzugt zusätzlich eine Vitamin-D-Komponente.

Extraossäre Kalzifizierung ist eine Ablagerung von Kalziumphosphatverbindungen in Weichteilgeweben außerhalb des Knochens. Es wird davon ausgegangen, dass zahlreiche Erkrankungen mit einer extraossären Kalzifizierung einhergehen bzw. auf diese zurückzuführen sind. Bei Patienten mit Nierenschädigungen bereitet die Kalzifizierung mitunter besondere Probleme, da diese Patienten besonders anfällig sind.

Die Dialysebehandlung übernimmt viele wichtige Aufgaben der Niere bei Patienten mit fortgeschrittener dialysepflichtiger Niereninsuffizienz, kann jedoch nicht alle Funktionen des natürlichen Organs vollständig ersetzen. Deshalb müssen chronisch Nierenkranke zusätzlich Medikamente einnehmen, um etwa den Mineralhaushalt des Körpers im Gleichgewicht zu halten und das Entstehen einer Blutarmut (Anämie) zu verhindern. Phosphat ist ein wichtiger Mineralstoff, der über die Nahrung aufgenommen wird und neben seiner Bedeutung für die Knochenbildung bei vielen physiologischen Prozessen eine wichtige Rolle spielt. Ein erhöhter Phosphatspiegel kann jedoch auch negative Folgen haben. Bei gesunden Menschen wird überschüssiges Phosphat über die Nieren ausgeschieden. Bei chronisch Nierenkranken verbleibt es dagegen im Blut und führt zu erhöhten Phosphatspiegeln, die mittels Dialyse nur begrenzt korrigiert werden können.

Die Knochenbildung ist ein stark regulierter Prozess. Er wird zum einen von Faktoren, die die Verknöcherung erleichtern, und zum anderen von solchen, die die Verknöcherung verhindern, reguliert. Dieses Gleichgewicht von Kalzifizierungsfaktoren und Kalzifizierungsinhibitoren führt letztendlich dazu, dass bei Gesunden eine ausreichende Mineralisierung des Knochens gewährleistet ist und andererseits Weichteilgewebe nicht verknöchern, bzw. kalzifizieren.

Bei einigen Erkrankungen kann es zu einem Ungleichgewicht zwischen den kalzifizierungsfördernden und den anti-kalzifizierenden Faktoren kommen. Insbesondere ist hier die terminale Niereninsuffizienz zu nennen. Hier führt unter anderem die Kombination aus erhöhten Phosphatspiegeln und einem Mangel an (funktionsfähigen) Kalzifizierungsinhibitoren zu einer vermehrten Ablagerung von Kalziumphosphatverbindungen in Weichteilgeweben, insbesondere in den verschiedenen Schichten der Blutgefäße (Arteriosklerose) aber auch in Organen, wie dem Herzen, seinen Gefäßen und Klappen und anderen Bereichen, wie in den Weichteilen.

Die Kalzifizierung der verschiedenen Organe kann zu einer Beeinträchtigung ihrer Funktion führen. So führt die Gefäßverkalkung zu einer Versteifung der Gefäße, die mit einer Blutdrucksteigerung und entsprechenden Endorganschäden einhergeht. Die Verkalkung der Herzklappen führt zu einer Beeinträchtigung ihrer Funktion.

Bei terminal niereninsuffizienten Patienten, aber auch bei Patienten mit anderen Erkrankungen, konnte gezeigt werden, dass eine extraossäre Kalzifizierung mit einem erhöhten Risiko für das Erleiden kardiovaskulärer Ereignisse wie Herzinfarkt und Schlaganfall assoziiert ist. Das Ausmaß der extraossären Kalzifizierung ist ein wichtiger Risikofaktor für Morbidität und Mortalität dieser Patienten. Eine aus medizinischer Sicht befriedigende Prävention oder Behandlung einer extraossären Kalzifizierung ist derzeit nicht möglich.

Y. Seyama et al., Internat. J. Vit. Nutr. Res. 66 (1996) 36-38 offenbart den Einfluss von Vitamin K₂ auf die Kalziumkonzentration im Serum und die Phosphatkonzentration im Serum von Ratten, bei denen eine vaskuläre Kalzifizierung mit Hilfe von Vitamin D₂ induziert wurde.

L.J. Schurgers et al., Blood, 2007, 109(7), 2823-31 offenbart, dass die Verabreichung hoher Dosen von Vitamin K bei Ratten eine durch Warfarin induzierte vaskuläre Kalzifizierung verringert.

L.J. Schurgers et al., Thromb. Haemost., 100, 2008, 593-603 offenbart u.a. die Verwendung von Vitamin K zur Reduzierung der Kalzifizierung von Blutgefäßen in Ratten.

M. K. Shea et al., Am J Clin Nutr 2009, 89, 1-9 betrifft eine klinische Studie, wonach Vitamin K₁ (500 µg/d) einer Kalzifizierung entgegenwirkt.

DE 885 992 offenbart ein Pflanzenmittel zur Herabsetzung der durch Frostschäden entstehenden Ernteverluste bei fruchttragenden Pflanzen, welches neben einem Pflanzenhormon eine Verbindung der Vitamin-K-Gruppe enthält. Als weitere aktive Substanz kann das Mittel Nikotinsäure oder ein Derivat dieser Säure enthalten, wie z.B. Nikotinamid. Zusammensetzungen, welche in Bezug auf die Reinheit und physiologische Verträglichkeit aller Inhaltsstoffe den allgemeinen arzneimittelrechtlichen Anforderungen für pharmazeutische Zusammensetzungen genügen und kein Pflanzenhormon enthalten, werden nicht offenbart.

DE 10 2007 003 524 offenbart, dass Nikotinsäureamid die zelluläre Aufnahme von Phosphat und die Akkumulation von Calciumphosphat in der Zelle hemmt. Dadurch wird die Gefahr reduziert, dass das Löslichkeitsprodukt von Calciumphosphat überschritten wird, Calciumphosphat ausfällt und die Gefahr von Arteriosklerose und das Auftreten verkalkter arteriosklerotischer Plaques vermindert wird.

EP-A 1 728 507 offenbart u.a. die Verwendung von Vitamin K zur Reduzierung oder Umkehrung der Kalzifizierung von Blutgefäßen.

US 2009/0192201 offenbart pharmazeutische Zusammensetzungen zur Behandlung und Vorbeugung von muskoloskeletalen und Bindegewebs-Erkrankungen unbekannter Ätiologie. Die Zusammensetzungen enthalten Vitamin K oder ein Analog von Vitamin K.

US 2009/0074883 offenbart Zusammensetzungen zur oralen Verabreichung von Nahrungsergänzungsstoffen. Die Zusammensetzungen können zahlreiche Vitamine enthalten, u.a. auch D, K oder B₃.

US 2008/317725 offenbart Zusammensetzungen, die verschiedene Vitamine enthalten und zur Nahrungsergänzung für Patienten mit Nierenerkrankungen, die unter einem Mangel an Nährstoffen leiden, verwendet werden.

XP 022797478 (WALLIN R. ET AL.: "Effects of the blood coagulation vitamin K as an inhibitor of arterial calcification", THROMBOSIS RESEARCH, Tarrytown, NY, US, Bd. 122, Nr. 3, (2008), S. 411-417) offenbart, dass ein Vitamin-K-abhängiges Protein namens MGP die Umwandlung von Zellen in den Gefäßwänden, die der Verkalkung von Gefäßen vorausgeht, hemmen kann.

XP 002657394 (EIJI SAITO ET AL.: "Treatment with vitamin K2 combined with biphosphonates synergistically inhibits calcification in cultured smooth muscle cells", JOURNAL OF ATHEROSCLEROSIS AND THROMBOSIS, Band 14, Nr.: 6, S.317-324, 01.12.2007 (2007)) offenbart, dass Vitamin K₂ alleine oder in Kombination mit einem Biphosphonat die Kalzifizierung in Arterien verringern kann.

XP 002657396 (KRUEGER THILO ET AL.: "Vitamin K deficiency in CKD patients: a modifiable risk factor for vascular calcification?", KIDNEY INTERNATIONAL, 01.07.2009 (2009), Band 76, S. 18-22) offenbart, dass es Hinweise darauf gibt, dass die arterielle Verkalkung bei Patienten mit einer chronischen Nierenkrankheit durch einen Mangel an Vitamin K hervorgerufen sein kann.

Es besteht daher ein Bedarf an Arzneimitteln, welche einer extraossären Kalzifizierung vorbeugen oder ihre Progredienz verzögern und sich somit zur Vorbeugung oder Behandlung von Erkrankungen eignen, die mit einer extraossären Kalzifizierung einhergehen.

Es ist eine Aufgabe der Erfindung, Arzneimittel bereitzustellen, welche Vorteile gegenüber den Arzneimitteln des Standes der Technik aufweisen.

Diese Aufgabe wird durch den Gegenstand der Patentansprüche gelöst.

Es wurde überraschend gefunden, dass durch kombinierte Verabreichung (simultan oder sequentiell) von einer Vitamin-K-Komponente und einer Nikotinamid-Komponente die Kalzifizierung signifikant vermindert werden kann. Durch die kombinierte Verabreichung (simultan oder sequentiell) von einer Vitamin-K-Komponente und einer Nikotinamid-Komponente können daher Erkrankungen vorgebeugt oder solche behandelt werden, welche mit einer Kalzifizierung einhergehen.

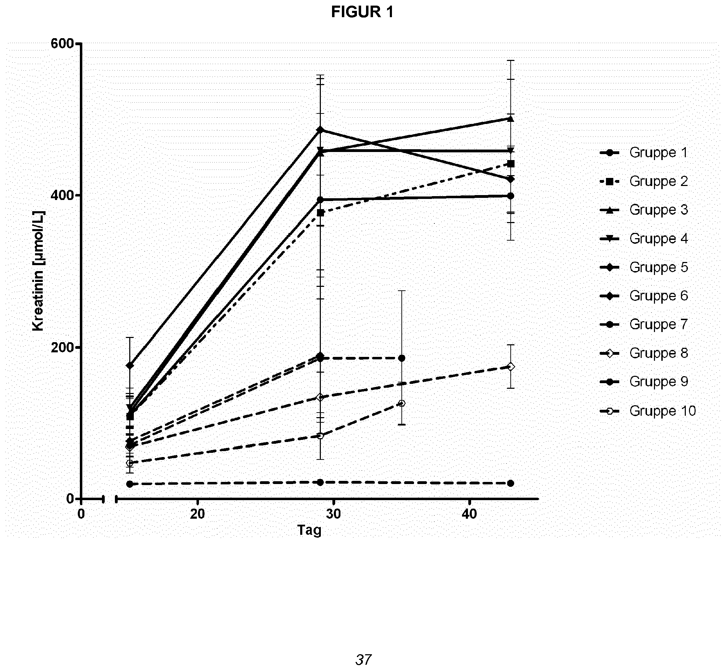

FIGUR 1 zeigt den Verlauf der Konzentration von Kreatinin im Plasma. Die Fehlerbalken zeigen die Standardabweichung.

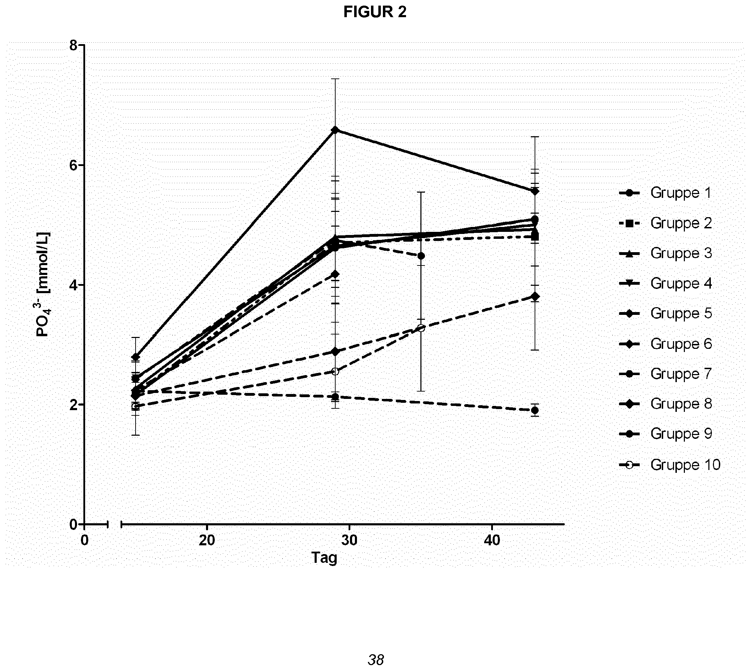

FIGUR 2 zeigt den Verlauf der Konzentration von Phosphat im Plasma. Die Fehlerbalken zeigen die Standardabweichung.

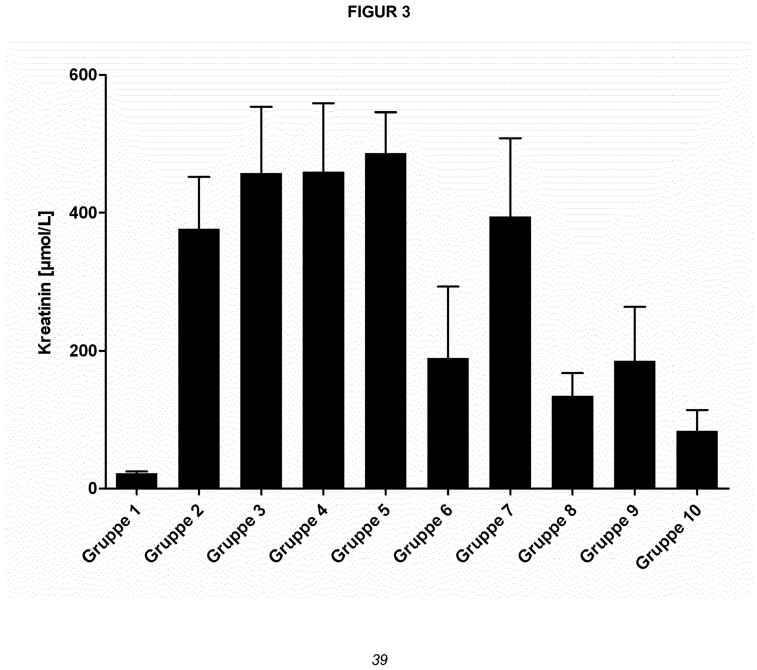

FIGUR 3 zeigt die Konzentration von Kreatinin im Plasma nach vierwöchiger Behandlung (Tag 29). Die Fehlerbalken zeigen die Standardabweichung.

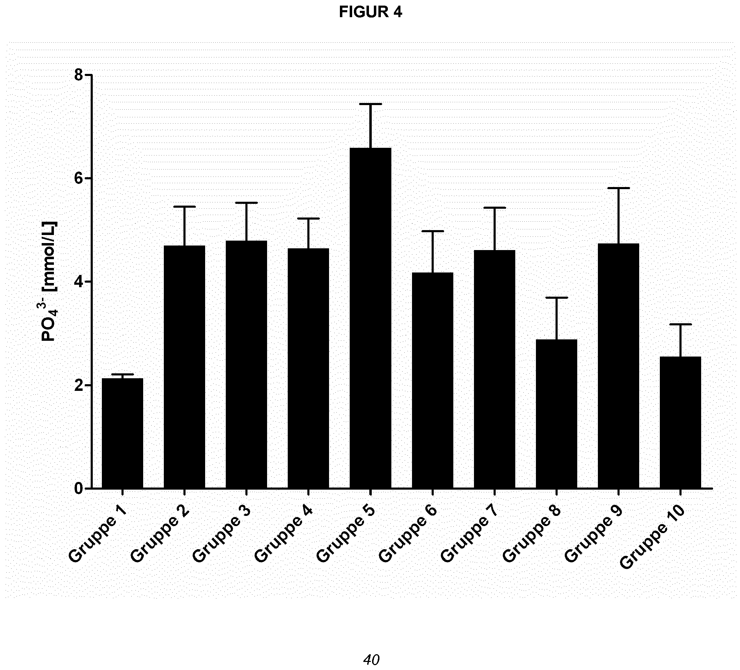

FIGUR 4 zeigt die Konzentration von Phosphat im Plasma nach vierwöchiger Behandlung (Tag 29). Die Fehlerbalken zeigen die Standardabweichung.

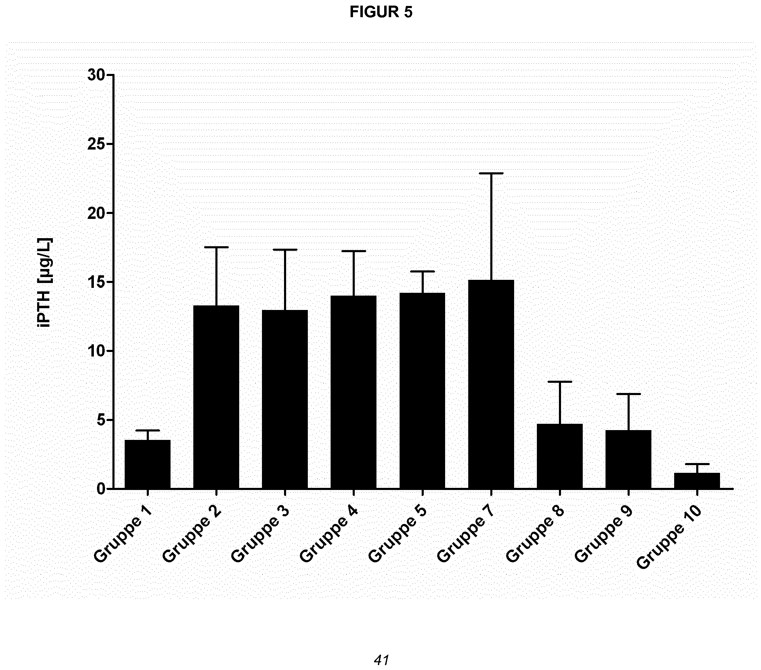

FIGUR 5 zeigt die Konzentration von iPTH (intaktes Parathyroid-Hormon) im Serum nach vierwöchiger Behandlung (Tag 29). Die Fehlerbalken zeigen die Standardabweichung.

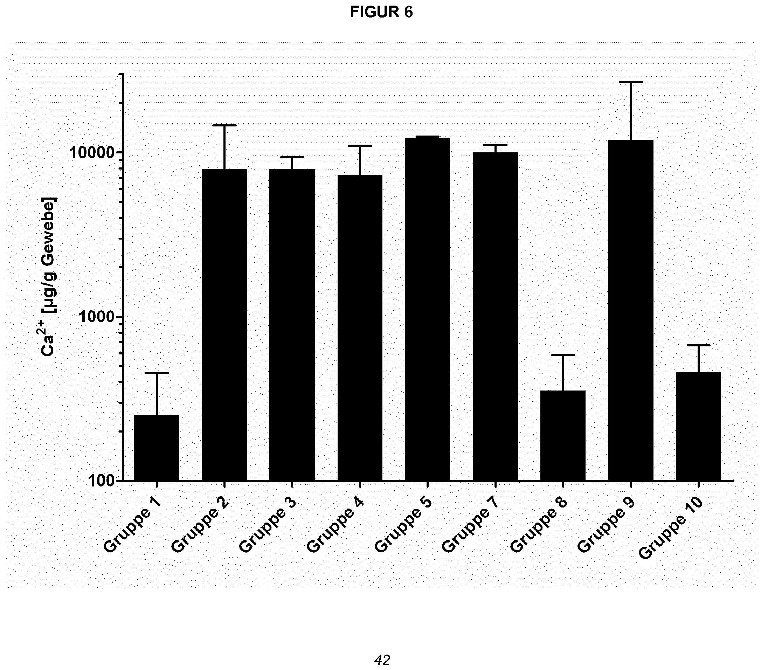

FIGUR 6 zeigt den Kalziumgehalt der Femoralarterie bei Nekropsie nach sechswöchiger Behandlung (Tag 43). Die Fehlerbalken zeigen die Standardabweichung.

Ein erster Aspekt der Erfindung betrifft eine pharmazeutische Zusammensetzung umfassend eine Vitamin-K-Komponente, ausgewählt aus der Gruppe bestehend aus Vitamin K₁, Vitamin K₂, Vitamin K₃, und Menadioldiphosphat, und eine Nikotinamid-Komponente, ausgewählt aus der Gruppe bestehend aus Nikotinsäure, Nikotinamid, L-Tryptophan, NAD, NAD⁺, NADH, NADP, NADP⁺, NADPH, Acipimox, Niceritrol und Nicorandil, wobei der Anteil der Vitamin-K-Komponente und der Nikotinamid-Komponente insgesamt wenigstens 10 Gew.-% beträgt, bezogen auf das Gesamtgewicht aller physiologisch aktiven Inhaltsstoffe der Zusammensetzung, und wobei das relative Gewichtsverhältnis der Vitamin-K-Komponente zur Nikotinamid-Komponente im Bereich von 1: 5 bis 1: 25.000 liegt, zur Anwendung bei der Vorbeugung oder Behandlung einer Erkrankung, welche mit einer extraossären Kalzifizierung einhergeht.

Bevorzugt beträgt der Anteil der Vitamin-K-Komponente und der Nikotinamid-Komponente insgesamt wenigstens 20 Gew.-%, bevorzugter wenigstens 30 Gew.-%, noch bevorzugter wenigstens 40 Gew.-%, am bevorzugtesten wenigstens 50 Gew.-% und insbesondere wenigstens 60 Gew.-%, bezogen auf das Gesamtgewicht aller physiologisch aktiven Inhaltsstoffe der Zusammensetzung.

In einer bevorzugten Ausführungsform beträgt der Anteil der Vitamin-K-Komponente und der Nikotinamid-Komponente insgesamt wenigstens 70 Gew.-% oder wenigstens 75 Gew.-%, bevorzugter wenigstens 80 Gew.-% oder wenigstens 85 Gew.-%, noch bevorzugter wenigstens 90 Gew.-% oder wenigstens 92 Gew.-%, am bevorzugtesten wenigstens 94 Gew.-% oder wenigstens 96 Gew.-% und insbesondere wenigstens 98 Gew.-% oder wenigstens 99 Gew.-%, bezogen auf das Gesamtgewicht aller physiologisch aktiven Inhaltsstoffe der Zusammensetzung.

Zum Zwecke der Beschreibung sind unter physiologisch aktiven Inhaltsstoffen alle solchen Stoffe zu verstehen, welche als Arzneistoffe, Nährstoffe, Nahrungsmittel oder Nahrungsergänzungsmittel klassifiziert werden können. Typische Hilfsstoffe pharmazeutischer Formulierungen, welche üblicherweise lediglich galenischen Zwecken dienen, sind erfindungsgemäß nicht als physiologisch aktive Inhaltsstoffe aufzufassen. Typische Vertreter physiologisch aktiver Inhaltsstoffe sind pharmakologisch aktive Substanzen (Arzneistoffe), Vitamine, Provitamine, Mineralstoffe, Spurenelemente, Enzyme, Fettsäuren, Aminosäuren, Antioxidantien, etc.

Das Gewicht aller physiologisch aktiven Inhaltsstoffe dient als Bezugsgröße für den prozentualen Anteil der Vitamin-K-Komponente und der Nikotinamid-Komponente, nicht jedoch das Gewicht der pharmazeutischen Zusammensetzung. Enthält die pharmazeutische Zusammensetzung beispielsweise insgesamt 43 Gew.-% physiologisch aktive Substanzen, bezogen auf das Gesamtgewicht der Zusammensetzung, so müssen die Vitamin-K-Komponente und die Nikotinamid-Komponente in ihrer Summe wenigstens 10 Gew.-% dieser 43 Gew.-% ausmachen, d.h. mindestens 4,3 Gew.-% bezogen auf das Gesamtgewicht der Zusammensetzung.

Zum Zwecke der Beschreibung umfasst der Begriff "Nikotinamid-Komponente" Nikotinsäure, Nikotinamid (Vitamin B₃), L-Tryptophan, NAD, NAD⁺, NADH, NADP, NADP⁺, NADPH, Acipimox, Niceritrol, Nicorandil und deren physiologisch verträgliche Salze bzw. Mischungen.

Zum Zwecke der Beschreibung umfasst der Begriff "Vitamin-K-Komponente" Vitamin K₁ (Phytomenadion), Vitamin K₂ (d.h. alle Menachinone), Vitamin K₃ (Menadion), und/oder Menadioldiphosphat und deren physiologisch verträgliche Salze bzw. Mischungen.

Weitere Referenz-Beispiele Vitamin-K-artig wirksamer Substanzen sind 2-Methyl-4-amino-1-naphthol, Menadiol bis(dihydrogen phosphat), Phytonadione, Menachinone 6, Menadione Bisulfit, 2-Methyl-4-amino-1-naphthol hydrochlorid, Menadione Natrium Bisulfite, Menadiol Natrium Diphosphat, Menadiol, N-(4-Hydroxy-3-methyl-1-naphthyl)acetamid, Menachinon 7, Menachinon 8, Menachinon 9, Menachinon 10, Phytonadiol, Acetomenaphthon, Phytonadiol Diacetat, Menatetrenon, gamma-Hydroxyvitamin K, 2-Chlorophyllochinon, Menadiol Natrium Sulfat, Menadoxim, Menadion Dimethylpyrimidinol Bisulfit, Menadioneepoxid, Phytonadion Hydroperoxid, Phytonadion Oxid, Demethylmenachinon, 2,5-Dimethyl-2-(4,8,12-trimethyl-tridecyl)-2H-naphtho(1,2-b)pyran-6-ol, Menadion Semichinon, Tetrahydromenachinon, 3-Bromomethylmenadion, Menadiol Dibutyrat, Phylloquinon Aglycon I, Phyllochinon Aglycon II, Phyllochinon Aglycon III, Phytonadion Epoxid-1,4-diol, 3-Hydroxy-2,3-dihydrophytonadion, Osteocalcin, 2-Trifluoromethylmenachinon und 2-Trifluoromethylphyllochinon.

Bevorzugt bilden die Vitamin-K-Komponente und die Nikotin-Komponente kein Menadion-Nicotinamid Bisulfit, so dass letzteres erfindungsgemäß bevorzugt nicht umfasst ist.

Bevorzugte individualisierte Kombinationen bestimmter Vitamin-K-Komponenten und bestimmter Nikotinamid-Komponenten sind nachfolgend zusammengefasst:
- {Nikotinamid + Vitamin K₁}, {Nikotinamid + Vitamin K₂}, {Nikotinamid + Vitamin K₃}, {Nikotinamid + Menadioldiphosphat};
- {NAD + Vitamin K₁}, {NAD + Vitamin K₂}, {NAD + Vitamin K₃}, {NAD + Menadioldiphosphat};
- {NAD⁺ + Vitamin K₁}, {NAD⁺ + Vitamin K₂}, {NAD⁺ + Vitamin K₃}, {NAD⁺ + Menadioldiphosphat};
- {NADH + Vitamin K₁}, {NADH + Vitamin K₂}, {NADH + Vitamin K₃}, {NADH + Menadioldiphosphat};
- {NADP + Vitamin K₁}, {NADP + Vitamin K₂}, {NADP + Vitamin K₃}, {NADP + Menadioldiphosphat};
- {NADP⁺ + Vitamin K₁}, {NADP⁺ + Vitamin K₂}, {NADP⁺ + Vitamin K₃}, {NADP⁺ + Menadioldiphosphat};
- {NADPH + Vitamin K₁}, {NADPH + Vitamin K₂}, {NADPH + Vitamin K₃}, oder {NADPH + Menadioldiphosphat}.

Es wurde überraschend gefunden, dass die Vitamin-K-Komponente und die Nikotinamid-Komponente im Hinblick auf die Verringerung der extraossären, vorzugsweise kardialen oder vaskulären Kalzifizierung synergistisch (superadditiv) zusammenwirken können.

Vorzugsweise wird der absolute Gewichtsanteil der Vitamin-K-Komponente und der Nikotinamid-Komponente und das relative Gewichtsverhältnis der Vitamin-K-Komponente zur Nikotinamid-Komponente so gewählt, dass im Hinblick auf die Verringerung der extraossären, vorzugsweise kardialen oder vaskulären Kalzifizierung, eine solche synergistische Wirkung erzielt wird. Dies kann durch einfache Routineversuche ermittelt werden.

Bevorzugt enthält die erfindungsgemäße Zusammensetzung mehr Gew.-% und/oder mol.-% an Nikotinamid-Komponente als an Vitamin-K-Komponente.

Erfindungsgemäß liegt das relative Gewichtsverhältnis der Vitamin-K-Komponente zur Nikotinamid-Komponente im Bereich von 1 : 5 bis 1 : 25.000, bevorzugt 1 : 10 bis 1 : 10.000, bevorzugter 1 : 100 bis 1 : 8.000, noch bevorzugter 1 : 200 bis 1 : 7.000, am bevorzugtesten 1 : 300 bis 1 : 6000 und insbesondere 1 : 400 bis 1 : 5500.

Erfindungsgemäß liegt das relative Gewichtsverhältnis der Vitamin-K-Komponente zur Nikotinamid-Komponente im Bereich von 1 : 5 bis 1 : 25.000, bevorzugt 1 : 5 bis 1 : 10.000, bevorzugter 1 : 5 bis 1 : 1.000, noch bevorzugter 1 : 5 bis 1 : 500, am bevorzugtesten 1 : 5 bis 1 : 300 und insbesondere 1 : 5 bis 1 : 150.

In einer bevorzugten Ausführungsform der erfindungsgemäßen Zusammensetzung liegt das relative Gewichtsverhältnis der Vitamin-K-Komponente zur Nikotinamid-Komponente im Bereich von 1 : (500±50%), bevorzugter 1 : (500±40%), noch bevorzugter 1 : (500±30%), am bevorzugtesten 1 : (500±20%) und insbesondere 1 : (500±10%).

In einer anderen bevorzugten Ausführungsform der erfindungsgemäßen Zusammensetzung liegt das relative Gewichtsverhältnis der Vitamin-K-Komponente zur Nikotinamid-Komponente im Bereich von 1 : (1000±50%), bevorzugter 1 : (1000±40%), noch bevorzugter 1 : (1000±30%), am bevorzugtesten 1 : (1000±20%) und insbesondere 1 : (1000±10%).

In einer weiteren bevorzugten Ausführungsform der erfindungsgemäßen Zusammensetzung liegt das relative Gewichtsverhältnis der Vitamin-K-Komponente zur Nikotinamid-Komponente im Bereich von 1 : (1500±50%), bevorzugter 1 : (1500±40%), noch bevorzugter 1 : (1500±30%), am bevorzugtesten 1 : (1500±20%) und insbesondere 1 : (1500±10%).

In einer anderen bevorzugten Ausführungsform der erfindungsgemäßen Zusammensetzung liegt das relative Gewichtsverhältnis der Vitamin-K-Komponente zur Nikotinamid-Komponente im Bereich von 1 : (2000±50%), bevorzugter 1 : (2000±40%), noch bevorzugter 1 : (2000±30%), am bevorzugtesten 1 : (2000±20%) und insbesondere 1 : (2000±10%).

In einer anderen bevorzugten Ausführungsform der erfindungsgemäßen Zusammensetzung liegt das relative Gewichtsverhältnis der Vitamin-K-Komponente zur Nikotinamid-Komponente im Bereich von 1 : (2500±50%), bevorzugter 1 : (2500±40%), noch bevorzugter 1 : (2500±30%), am bevorzugtesten 1 : (2500±20%) und insbesondere 1 : (2500±10%).

In einer weiteren bevorzugten Ausführungsform der erfindungsgemäßen Zusammensetzung liegt das relative Gewichtsverhältnis der Vitamin-K-Komponente zur Nikotinamid-Komponente im Bereich von 1 : (3000±50%), bevorzugter 1 : (3000±40%), noch bevorzugter 1 : (3000±30%), am bevorzugtesten 1 : (3000±20%) und insbesondere 1 : (3000±10%).

In einer anderen bevorzugten Ausführungsform der erfindungsgemäßen Zusammensetzung liegt das relative Gewichtsverhältnis der Vitamin-K-Komponente zur Nikotinamid-Komponente im Bereich von 1 : (3500±50%), bevorzugter 1 : (3500±40%), noch bevorzugter 1 : (3500±30%), am bevorzugtesten 1 : (3500±20%) und insbesondere 1 : (3500±10%).

In einer weiteren bevorzugten Ausführungsform der erfindungsgemäßen Zusammensetzung liegt das relative Gewichtsverhältnis der Vitamin-K-Komponente zur Nikotinamid-Komponente im Bereich von 1 : (4000±50%), bevorzugter 1 : (4000±40%), noch bevorzugter 1 : (4000±30%), am bevorzugtesten 1 : (4000±20%) und insbesondere 1 : (4000±10%).

In einer anderen bevorzugten Ausführungsform der erfindungsgemäßen Zusammensetzung liegt das relative Gewichtsverhältnis der Vitamin-K-Komponente zur Nikotinamid-Komponente im Bereich von 1 : (4500±50%), bevorzugter 1 : (4500±40%), noch bevorzugter 1 : (4500±30%), am bevorzugtesten 1 : (4500±20%) und insbesondere 1 : (4500±10%).

In einer weiteren bevorzugten Ausführungsform der erfindungsgemäßen Zusammensetzung liegt das relative Gewichtsverhältnis der Vitamin-K-Komponente zur Nikotinamid-Komponente im Bereich von 1 : (5000±50%), bevorzugter 1 : (5000±40%), noch bevorzugter 1 : (5000±30%), am bevorzugtesten 1 : (5000±20%) und insbesondere 1 : (5000±10%).

In einer bevorzugten Ausführungsform beträgt der relative Gewichtsanteil der Nikotinamid-Komponente wenigstens 1,0 Gew.-%, bevorzugter wenigstens 5,0 Gew.-%, noch bevorzugter wenigstens 10 Gew.-%, am bevorzugtesten wenigstens 15 Gew.-% und insbesondere wenigstens 20 Gew.-%, bezogen auf das Gesamtgewicht der Zusammensetzung.

In einer bevorzugten Ausführungsform beträgt der relative Gewichtsanteil der Nikotinamid-Komponente höchstens 80 Gew.-%, bevorzugter höchstens 70 Gew.-%, noch bevorzugter höchstens 60 Gew.-%, am bevorzugtesten höchstens 50 Gew.-% und insbesondere höchstens 40 Gew.-%, bezogen auf das Gesamtgewicht der Zusammensetzung.

In einer bevorzugten Ausführungsform beträgt der relative Gewichtsanteil der Vitamin-K-Komponente wenigstens 0,001 Gew.-%, wenigstens 0,002 Gew.-% oder wenigstens 0,004 Gew.-%; bevorzugter wenigstens 0,006 Gew.-%, wenigstens 0,008 Gew.-% oder wenigstens 0,01 Gew.-%; noch bevorzugter wenigstens 0,02 Gew.-%, wenigstens 0,04 Gew.-% oder wenigstens 0,06 Gew.-%; am bevorzugtesten wenigstens 0,08 Gew.-%, wenigstens 0,1 Gew.-% oder wenigstens 0,2 Gew.-%; und insbesondere wenigstens 0,3 Gew.-% wenigstens 0,4 Gew.-% oder wenigstens 0,5 Gew.-%; bezogen auf das Gesamtgewicht der Zusammensetzung.

In einer bevorzugten Ausführungsform beträgt der relative Gewichtsanteil der Vitamin-K-Komponente höchstens 1,0 Gew.-%, höchstens 0,9 Gew.-% oder höchstens 0,8 Gew.%-; bevorzugter höchstens 0,7 Gew.-%, höchstens 0,6 Gew.-% oder höchstens 0,5 Gew.-%; noch bevorzugter höchstens 0,4 Gew.-%, höchstens 0,3 Gew.-% oder höchstens 0,2 Gew.-%; am bevorzugtesten höchstens 0,1 Gew.-%, höchstens 0,08 Gew.-% oder höchstens 0,06 Gew.-%; und insbesondere höchstens 0,04 Gew.-% höchstens 0,02 Gew.-% oder höchstens 0,01 Gew.-%; bezogen auf das Gesamtgewicht der Zusammensetzung.

Neben der Vitamin-K-Komponente und der Nikotinamid-Komponente kann die erfindungsgemäße pharmazeutische Zusammensetzung physiologisch verträgliche Hilfsstoffe enthalten. Geeignete Hilfsstoffe sind dem Fachmann grundsätzlich bekannt. In diesem Zusammenhang kann beispielsweise vollumfänglich verwiesen werden auf H.P. Fieder, *Lexikon der Hilfsstoffe für Pharmazie, Kosmetik und angrenzende technische Gebiete,* Editio Cantor Aulendorf.

Die erfindungsgemäße Zusammensetzung ist bevorzugt fest, halbfest, pastös oder flüssig.

Ist die erfindungsgemäße Zusammensetzung fest, so liegt sie vorzugsweise als loses Pulver, als Granulat oder in kompaktierter Form, z.B. in Form von Tabletten oder Pellets vor.

Ist die erfindungsgemäße Zusammensetzung halbfest, pastös oder flüssig, so liegt sie vorzugsweise als Lösung und/oder Dispersion der Vitamin-K-Komponente und/oder der Nikotinamid-Komponente vor, wobei die Dispersion eine Emulsion (O/W oder W/O) oder eine Dispersion sein kann. Als Lösungsmittel kommen insbesondere Wasser und andere physiologisch verträgliche Flüssigkeiten in Betracht, welche üblicherweise zur Herstellung halbfester, pastöser oder flüssiger Formulierungen eingesetzt werden.

Neben der Vitamin-K-Komponente und der Nikotinamid-Komponente kann die erfindungsgemäße pharmazeutische Zusammensetzung weitere physiologisch aktive Substanzen enthalten, beispielsweise weitere Vitamine oder auch Pyrophosphat.

In einer bevorzugten Ausführungsform enthält die erfindungsgemäße Zusammensetzung die Vitamin-K-Komponente und die Nikotinamid-Komponente als einzige physiologisch aktive Substanzen.

In einer bevorzugten Ausführungsform enthält die erfindungsgemäße Zusammensetzung kein Pflanzenhormon, vorzugsweise überhaupt kein Hormon.

In einer anderen bevorzugten Ausführungsform enthält die erfindungsgemäße Zusammensetzung kein Vitamin C und/oder keine mehrfach ungesättigten Fettsäuren.

In einer weiteren bevorzugten Ausführungsform enthält die erfindungsgemäße Zusammensetzung neben der Vitamin-K-Komponente, der Nikotinamid-Komponente und der ggf. vorhandenen Vitamin-D-Komponente keine weiteren Vitamine, vorzugsweise keine weiteren physiologisch oder pharmakologisch aktiven Inhaltsstoffe.

In einer anderen bevorzugten Ausführungsform enthält die erfindungsgemäße Zusammensetzung eine einzelne zusätzliche physiologisch aktive Substanz, so dass insgesamt (einschließlich der Vitamin-K-Komponente und der Nikotinamid-Komponente) drei physiologisch aktive Substanzen enthalten sind, vorzugsweise jedoch nicht mehr. Bevorzugt ist diese zusätzliche physiologisch aktive Substanz ein Vitamin, besonders bevorzugt eine Vitamin-D-Komponente.

Zum Zwecke der Beschreibung umfasst der Begriff "Vitamin-D-Komponente" alle Vitamin-D-artig wirksamen Substanzen, insbesondere Vitamin D₁, Vitamin D₂ (Ergocalciferol), Vitamin D₃ (Colecalciferol), 25-Hydroxy-colecaliferol, 1-Hydroxycolecalciferol, 1,25-Dihydroxycolecalciferol (Calcitriol), Dihydrotachysterol, 25-Hydroxyvitamin D₂, 1,25-Dihydroxyvitamin D₂, 1-Hydroxyvitamin D₂, Hectorol, Paricalcitol, etc., wobei Calcitriol besonders bevorzugt ist, sowie deren physiologisch verträglichen Salze bzw. Mischungen.

Bevorzugte individualisierte Kombinationen bestimmter Vitamin-K-Komponenten, bestimmter Nikotinamid-Komponenten und bestimmter Vitamin-D-Komponenten sind nachfolgend zusammengefasst:
- {Nikotinamid + Vitamin K₁ + Vitamin D₁}, {Nikotinamid + Vitamin K₂ + Vitamin D₁}, {Nikotinamid + Vitamin K₃ + Vitamin D₁}, {Nikotinamid + Menadioldiphosphat + Vitamin D₁};
- {NAD + Vitamin K₁ + Vitamin D₁}, {NAD + Vitamin K₂ + Vitamin D₁}, {NAD + Vitamin K₃ + Vitamin D₁}, {NAD + Menadioldiphosphat + Vitamin D₁};
- {NAD⁺ + Vitamin K₁ + Vitamin D₁}, {NAD⁺ + Vitamin K₂ + Vitamin D₁}, {NAD⁺ + Vitamin K₃ + Vitamin D₁}, {NAD⁺ + Menadioldiphosphat + Vitamin D₁};
- {NADH + Vitamin K₁ + Vitamin D₁}, {NADH + Vitamin K₂ + Vitamin D₁}, {NADH + Vitamin K₃ + Vitamin D₁}, {NADH + Menadioldiphosphat + Vitamin D₁};
- {NADP + Vitamin K₁ + Vitamin D₁}, {NADP + Vitamin K₂ + Vitamin D₁}, {NADP + Vitamin K₃ + Vitamin D₁}, {NADP + Menadioldiphosphat + Vitamin D₁};
- {NADP⁺ + Vitamin K₁ + Vitamin D₁}, {NADP⁺ + Vitamin K₂ + Vitamin D₁}, {NADP⁺ + Vitamin K₃ + Vitamin D₁}, {NADP⁺ + Menadioldiphosphat + Vitamin D₁};
- {NADPH + Vitamin K₁ + Vitamin D₁}, {NADPH + Vitamin K₂ + Vitamin D₁}, {NADPH + Vitamin K₃ + Vitamin D₁}, oder {NADPH + Menadioldiphosphat + Vitamin D₁};
   ∘ {Nikotinamid + Vitamin K₁ + Vitamin D₂}, {Nikotinamid + Vitamin K₂ + Vitamin D₂}, {Nikotinamid + Vitamin K₃ + Vitamin D₂}, {Nikotinamid + Menadioldiphosphat + Vitamin D₂};
   ∘ {NAD + Vitamin K₁ + Vitamin D₂}, {NAD + Vitamin K₂ + Vitamin D₂}, {NAD + Vitamin K₃ + Vitamin D₂}, {NAD + Menadioldiphosphat + Vitamin D₂};
   ∘ {NAD⁺ + Vitamin K₁ + Vitamin D₂}, {NAD⁺ + Vitamin K₂ + Vitamin D₂}, {NAD⁺ + Vitamin K₃ + Vitamin D₂}, {NAD⁺ + Menadioldiphosphat + Vitamin D₂};
   ∘ {NADH + Vitamin K₁ + Vitamin D₂}, {NADH + Vitamin K₂ + Vitamin D₂}, {NADH + Vitamin K₃ + Vitamin D₂}, {NADH + Menadioldiphosphat + Vitamin D₂};
   ∘ {NADP + Vitamin K₁ + Vitamin D₂}, {NADP + Vitamin K₂ + Vitamin D₂}, {NADP + Vitamin K₃ + Vitamin D₂}, {NADP + Menadioldiphosphat + Vitamin D₂};
   ∘ {NADP⁺ + Vitamin K₁ + Vitamin D₂}, {NADP⁺ + Vitamin K₂ + Vitamin D₂}, {NADP⁺ + Vitamin K₃ + Vitamin D₂}, {NADP⁺ + Menadioldiphosphat + Vitamin D₂};
   ∘ {NADPH + Vitamin K₁ + Vitamin D₂}, {NADPH + Vitamin K₂ + Vitamin D₂}, {NADPH + Vitamin K₃ + Vitamin D₂}, oder {NADPH + Menadioldiphosphat + Vitamin D₂};
      ▪ {Nikotinamid + Vitamin K₁ + Vitamin D₃}, {Nikotinamid + Vitamin K₂ + Vitamin D₃}, {Nikotinamid + Vitamin K₃ + Vitamin D₃}, {Nikotinamid + Menadioldiphosphat + Vitamin D₃};
      ▪ {NAD + Vitamin K₁ + Vitamin D₃}, {NAD + Vitamin K₂ + Vitamin D₃}, {NAD + Vitamin K₃ + Vitamin D₃}, {NAD + Menadioldiphosphat + Vitamin D₃};
      ▪ {NAD⁺ + Vitamin K₁ + Vitamin D₃}, {NAD⁺ + Vitamin K₂ + Vitamin D₃}, {NAD⁺ + Vitamin K₃ + Vitamin D₃}, {NAD⁺ + Menadioldiphosphat + Vitamin D₃};
      ▪ {NADH + Vitamin K₁ + Vitamin D₃}, {NADH + Vitamin K₂ + Vitamin D₃}, {NADH + Vitamin K₃ + Vitamin D₃}, {NADH + Menadioldiphosphat + Vitamin D₃};
      ▪ {NADP + Vitamin K₁ + Vitamin D₃}, {NADP + Vitamin K₂ + Vitamin D₃}, {NADP + Vitamin K₃ + Vitamin D₃}, {NADP + Menadioldiphosphat + Vitamin D₃};
      ▪ {NADP⁺ + Vitamin K₁ + Vitamin D₃}, {NADP⁺ + Vitamin K₂ + Vitamin D₃}, {NADP⁺ + Vitamin K₃ + Vitamin D₃}, {NADP⁺ + Menadioldiphosphat + Vitamin D₃};
      ▪ {NADPH + Vitamin K₁ + Vitamin D₃}, {NADPH + Vitamin K₂ + Vitamin D₃}, {NADPH + Vitamin K₃ + Vitamin D₃}, oder {NADPH + Menadioldiphosphat + Vitamin D₃}.

Enthält die erfindungsgemäße Zusammensetzung eine Vitamin-D-Komponente, liegt das relative Gewichtsverhältnis der Vitamin-D-Komponente zur Nikotinamid-Komponente in einer bevorzugten Ausführungsform im Bereich von 1 : (25.000±50%), bevorzugter 1 : (25.000±40%), noch bevorzugter 1 : (25.000±30%), am bevorzugtesten 1 : (25.000±20%) und insbesondere 1 : (25.000±10%).

In einer anderen bevorzugten Ausführungsform liegt das relative Gewichtsverhältnis der Vitamin-D-Komponente zur Nikotinamid-Komponente im Bereich von 1 : (100.000±50%), bevorzugter 1 : (100.000±40%), noch bevorzugter 1 : (100.000±30%), am bevorzugtesten 1 : (100.000±20%) und insbesondere 1 : (100.000±10%).

In einer weiteren bevorzugten Ausführungsform liegt das relative Gewichtsverhältnis der Vitamin-D-Komponente zur Nikotinamid-Komponente im Bereich von 1 : (500.000±50%), bevorzugter 1 : (500.000±40%), noch bevorzugter 1 : (500.000±30%), am bevorzugtesten 1 : (500.000±20%) und insbesondere 1 : (500.000±10%).

In einer weiteren bevorzugten Ausführungsform liegt das relative Gewichtsverhältnis der Vitamin-D-Komponente zur Nikotinamid-Komponente im Bereich von 1 : 1.000.000 bis 1 : 1.000, bevorzugter 1 : 500.000 bis 1 : 5.000, noch bevorzugter 1 : 400.000 bis 1 : 10.000, am bevorzugtesten 1 : 300.000 bis 1 : 15.000 und insbesondere 1 : 200.000 bis 1 : 20.000.

In einer bevorzugten Ausführungsform liegt das relative Gewichtsverhältnis der Vitamin-D-Komponente zur Vitamin-K-Komponente im Bereich von 1 : 5 bis 1 : 25.000, bevorzugter 1 : 10 bis 1 : 10.000, bevorzugter 1 : 100 bis 1 : 8.000, noch bevorzugter 1 : 200 bis 1 : 7.000, am bevorzugtesten 1 : 300 bis 1 : 6000 und insbesondere 1 : 400 bis 1 : 5500.

In einer bevorzugten Ausführungsform liegt das relative Gewichtsverhältnis der Vitamin-D-Komponente zur Vitamin-K-Komponente im Bereich von 1 : (1.000±50%), bevorzugter 1 : (1.000±40%), noch bevorzugter 1 : (1.000±30%), am bevorzugtesten 1 : (1.000±20%) und insbesondere 1 : (1.000±10%).

Bevorzugte Ausführungsformen A¹ bis A⁷ der erfindungsgemäßen Zusammensetzung, welche neben der Nikotinamid-Komponente und der Vitamin-K-Komponente zusätzlich eine Vitamin-D-Komponente umfasst, sind in nachfolgender Tabelle zusammengefasst. Dabei bedeutet N ein Gewichtsäquivalent der Nikotinamid-Komponente, K ein Gewichtsäquivalent der Vitamin-K-Komponente und D ein Gewichtsäquivalent der Vitamin-D-Komponente, so dass das Verhältnis N : K : D das relative Gewichtsverhältnis aller drei Komponenten zueinander definiert:

| N : K : D | A¹ | A² | A³ | A⁴ | A⁵ | A⁶ | A⁷ |
|---|---|---|---|---|---|---|---|
| N | 10^{(6±80%)} | 10^{(6±60%)} | 10^{(6±50%)} | 10^{(6±40%)} | 10^{(6±30%)} | 10^{(6±20%)} | 10^{(6±10%)} |
| K | 10^{(3±80%)} | 10^{(3±60%)} | 10^{(3±50%)} | 10^{(3±40%)} | 10^{(3±30%)} | 10^{(3±20%)} | 10^{(3±10%)} |
| D | 1 | 1 | 1 | 1 | 1 | 1 | |

Demzufolge liegt beispielsweise gemäß Ausführungsform A³ das Verhältnis N : K : D im Bereich von (10⁹ ≥ N ≥ 10³) : (10^{4,5} ≥ K ≥ 10^{1,5}) : D.

Ein weiterer Aspekt der Erfindung betrifft eine pharmazeutische Darreichungsform, welche die vorstehend beschriebene pharmazeutische Zusammensetzung umfasst, zur Anwendung bei der Vorbeugung oder Behandlung einer Erkrankung, welche mit einer extraossären Kalzifizierung einhergeht. Bevorzugte Ausführungsformen der erfindungsgemäßen pharmazeutischen Zusammensetzung gelten entsprechend auch für die erfindungsgemäße pharmazeutische Darreichungsform.

Zum Zwecke der Beschreibung ist der Begriff "Darreichungsform" vorzugsweise synonym zu "Medikament", "Arzneiform" bzw. "Dosiseinheit". Handelt es sich z.B. um ein Medikament zur oralen Verabreichung, beispielsweise in Form einer Tablette, so stellt diese Tablette vorzugsweise die zu verabreichende Dosiseinheit dar, welche die für den jeweiligen Zeitpunkt der Verabreichung innerhalb eines Behandlungsschemas vorgesehene Dosis der Vitamin-K-Komponente und der Nikotinamid-Komponente enthält. Umfasst die Dosiseinheit eine einzige Tablette, so entspricht die Dosiseinheit der Darreichungsform. Es ist jedoch auch möglich, dass die Dosiseinheit auf mehrere Darreichungsformen aufgeteilt ist, beispielsweise mehrere Tabletten, welche jeweils nur eine Teildosis, in der Gesamtheit jedoch die Gesamtdosis der Vitamin-K-Komponente und der Nikotinamid-Komponente enthalten, die für den jeweiligen Zeitpunkt der Verabreichung innerhalb eines Behandlungsschemas vorgesehen ist (diese Tabletten der Dosiseinheit sind dann zur im wesentlichen gleichzeitigen Verabreichung vorgesehen).

In einer bevorzugten Ausführungsform ist die erfindungsgemäße Darreichungsform zur oralen oder parenteralen Verabreichung konfektioniert.

In einer bevorzugten Ausführungsform ist die erfindungsgemäße Darreichungsform zur parenteralen Verabreichung konfektioniert. Parenterale Verabreichungen können beispielsweise erfolgen subkutan, intravenös, intramuskulär, intraarteriell, intraperitoneal, intrakutan, intraartikulär, intrathekal, intrakardial, intravitreal, retrobulbär, intrapulmonal und intraossär.

Besonders bevorzugt ist die erfindungsgemäße Darreichungsform zur Injektion oder Infusion konfektioniert, insbesondere zur intravenösen oder intraarteriellen Verabreichung.

Geeignete Darreichungsformen, welche zur Injektion bzw. Infusion geeignet sind, sind dem Fachmann bekannt. In diesem Zusammenhang kann beispielsweise vollumfänglich verwiesen werden auf K.H. Bauer et al., Lehrbuch der Pharmazeutischen Technologie, WVG Stuttgart 1999.

Darreichungsformen, welche zur Injektion geeignet sind, sind üblicherweise sterile Lösungen, Emulsionen oder Suspensionen, welche durch Lösen, Emulgieren oder Suspendieren der aktiven Substanz und gegebenenfalls weiterer Hilfsstoffe, ggf. als Lyophilisate, in Wasser, in einer geeigneten nichtwässrigen Flüssigkeit, welche nicht steril sein muss, falls dies gerechtfertigt ist, oder in einer Mischung dieser Vehikel hergestellt werden.

Darreichungsformen, welche zur Infusion geeignet sind, sind üblicherweise sterile, wässrige Lösungen oder Emulsionen mit Wasser als kontinuierlicher Phase.

Darreichungsformen zur Injektion bzw. Infusion können ggf. weitere Hilfsstoffe enthalten. Solche Hilfsstoffe sind vorzugsweise Löslichkeitsvermittler wie beispielsweise Lecithin und Poloxamer 188, Stoffe zur Isotonoisierung wie beispielsweise Natriumchlorid, Glucose und Mannit, Puffer wie beispielsweise Acetat-, Phosphat- und Citratpuffer, Antioxidantien wie beispielsweise Ascorbinsäure, Natriummetahydrogensulfit, Natriumsulfit und Natriumhydrogensulfit, Chelatbildner wie beispielsweise Dinatriumedetat, Konservierungsmittel wie beispielsweise p-Hydroxybenzoesäureester, Benzylalkohol und Chlorkresol und Emulgatoren wie beispielsweise Lecithin, Fettalkohole, Sterole, Sorbitanfettsäureester, PolyoxyethylenSorbitanfettsäureester, Polyoxyethylen-Fettsäureglyceride, Polyoxyethylen-Fettsäureester, Polyoxyethylen-Fettalkoholether, Glycerolfettsäureester und Poloxamere.

In einer anderen bevorzugten Ausführungsform ist die erfindungsgemäße Darreichungsform zur oralen Verabreichung konfektioniert. Bevorzugt ist die erfindungsgemäße Darreichungsform eine orale Darreichungsform ausgewählt aus der Gruppe bestehend aus Tabletten, Kapseln, Dragees, Pellets und Sachets.

Geeignete Darreichungsformen, welche zur oralen Verabreichung geeignet sind (orale Medikamente), sind dem Fachmann bekannt. In diesem Zusammenhang kann beispielsweise vollumfänglich verwiesen werden auf K.H. Bauer et al., Lehrbuch der Pharmazeutischen Technologie, WVG Stuttgart 1999.

Die orale Darreichungsform wird bevorzugt ausgewählt aus der Gruppe bestehend aus Tabletten, Pulvern, Pellets, Granulaten, Dragees, Sirupen, Säften, Lösungen, Brausepulvern, Brausegranulaten, Brausetabletten, Lyophilisaten und Kapseln. Besonders bevorzugt ist die orale Darreichungsform eine Tablette, Filmtablette, ein Dragee, Granulat, Pellet oder Pulver, besonders bevorzugt eine Tablette.

Geeignete Hilfsstoffe zur Formulierung oraler Darreichungsformen sind dem Fachmann bekannt. In diesem Zusammenhang kann beispielsweise verwiesen werden auf H.P. Fiedler, Lexikon der Hilfsstoffe für Pharmazie, Kosmetik und angrenzende Gebiete, Editio Cantor Aulendorf, 2001.

Tabletten können beispielsweise durch Mischen der Vitamin-K-Komponente und der Nikotinamid-Komponente mit bekannten Hilfsstoffen, beispielsweise inerten Verdünnungsmitteln, wie Kalziumcarbonat, Kalziumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen. Die Tabletten können außer den genannten Trägerstoffen auch Zusätze, wie z.B. Natriumcitrat, Kalziumcarbonat und Dikalziumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden.

Dragees können z.B. durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Auch kann die Drageehülle zur Erzielung eines Depoteffektes aus mehreren Schichten bestehen wobei die oben bei den Tabletten erwähnten Hilfsstoffe verwendet werden können.

Säfte, Sirupe, Emulsionen, Suspensionen und Lösungen zur oralen Applikation können zusätzlich noch ein Süßungsmittel, wie Saccharin, Cyclamat, Glycerin oder Zucker sowie ein den Geschmack verbesserndes Mittel, beispielsweise Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Konservierungsmittel, wie p-Hydroxybenzoesäureester, enthalten.

Kapseln können beispielsweise hergestellt werden, indem man die der Vitamin-K-Komponente und die Nikotinamid-Komponente mit inerten Trägern, wie Milchzucker oder Sorbit, mischt und in Gelatinekapseln einkapselt. Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie beispielsweise natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Das Medikament kann die Vitamin-K-Komponente und/oder die Nikotinamid-Komponente sofort oder kontrolliert freisetzen. Erfolgt die Freisetzung kontrolliert, so erfolgt die Freisetzung vorzugsweise retardiert. Unter retardierter Freisetzung wird erfindungsgemäß bevorzugt ein Freisetzungsprofil verstanden, bei dem die Vitamin-K-Komponente und/oder die Nikotinamid-Komponente mit dem Ziel einer verlängerten therapeutischen Wirkung über einen längeren Zeitraum bei verringerter Einnahmefrequenz freigegeben wird. Insbesondere wird dies bei oraler Verabreichung erreicht. Der Ausdruck "mit zumindest teilweise retardierter Freisetzung" umfasst erfindungsgemäß jegliche Darreichungsform, welche eine modifizierte Freigabe der darin enthaltenen Vitamin-K-Komponente und/oder Nikotinamid-Komponente gewährleistet.

Bei den Darreichungsformen handelt es sich bevorzugt um überzogene oder nicht überzogene Darreichungsformen, die mit speziellen Hilfsstoffen, nach besonderen Verfahren oder durch Kombination beider Möglichkeiten hergestellt werden, um die Freisetzungsgeschwindigkeit oder den Ort der Freisetzung gezielt zu verändern. Hinsichtlich des zeitlichen Ablaufs der Freigabe sind bei den erfindungsgemäßen Medikamenten folgende Arten umfasst: verzögerte Freigabe *(extended release, delayed release),* gestaffelte Freigabe *(repeat action release),* hinhaltende Freigabe *(prolonged release)* und gleichmäßig hinhaltende Freigabe (*sustained rele*ase). Hinsichtlich weiterer Einzelheiten kann beispielsweise verwiesen werden auf K.H. Bauer, Lehrbuch der Pharmazeutischen Technologie, 6. Auflage, WVG Stuttgart, 1999.

Geeignete Maßnahmen zur kontrollierten Wirkstofffreisetzung sind dem Fachmann bekannt. Handelt es sich bei dem Medikament um eine orale Darreichungsform, beispielsweise eine Tablette, so kann eine verzögerte Freisetzung beispielsweise durch Einbettung der Vitamin-K-Komponente und/oder der Nikotinamid-Komponente in einer Polymermatrix und/oder Filmbeschichtung der oralen Darreichungsform mit einer Membran erreicht werden.

Bevorzugt ist die Darreichungsform zur einmal, zweimal oder dreimal täglichen Verabreichung konfektioniert. In einer bevorzugten Ausführungsform ist die Darreichungsform zur dreimal wöchentlichen Dialyse konfektioniert.

In einer bevorzugten Ausführungsform enthält die Darreichungsform die Vitamin-K-Komponente bevorzugt in einer Dosis von wenigstens 0,01 mg, wenigstens 0,10 mg, wenigstens 0,25 mg, wenigstens 0,50 mg, wenigstens 0,75 mg, wenigstens 1,00 mg, wenigstens 1,25 mg, wenigstens 1,50 mg, wenigstens 1,75 mg oder wenigstens 2,00 mg; bevorzugter wenigstens 2,25 mg, wenigstens 2,50 mg, wenigstens 2,75 mg, wenigstens 3,00 mg, wenigstens 3,25 mg, wenigstens 3,50 mg, wenigstens 3,75 mg oder wenigstens 4,00 mg; noch bevorzugter wenigstens 4,25 mg, wenigstens 4,50 mg, wenigstens 4,75 mg, wenigstens 5,00 mg, wenigstens 5,25 mg, wenigstens 5,50 mg, wenigstens 5,75 mg oder wenigstens 6,00 mg; am bevorzugtesten wenigstens 6,25 mg, wenigstens 6,50 mg, wenigstens 6,75 mg, wenigstens 7,00 mg, wenigstens 7,25 mg, wenigstens 7,50 mg, wenigstens 7,75 mg oder wenigstens 8,00 mg; und insbesondere wenigstens 8,25 mg, wenigstens 8,50 mg, wenigstens 8,75 mg, wenigstens 9,00 mg, wenigstens 9,25 mg, wenigstens 9,50 mg, wenigstens 9,75 mg oder wenigstens 10,00 mg; bevorzugt jeweils als Äquivalentdosis bezogen auf Vitamin K₁.

In einer bevorzugten Ausführungsform enthält die Darreichungsform die Vitamin-K-Komponente bevorzugt in einer Dosis von höchstens 100 mg, höchstens 97,50 mg, höchstens 95,00 mg, höchstens 92,50 mg, höchstens 90,00 mg, höchstens 87,50 mg, höchstens 85,00 mg oder höchstens 82,50 mg; bevorzugter höchstens 80,00 mg, höchstens 77,50 mg, höchstens 75,00 mg, höchstens 72,50 mg, höchstens 70,00 mg, höchstens 67,50 mg, höchstens 65,00 mg oder höchstens 62,50 mg; noch bevorzugter höchstens 60,00 mg, höchstens 57,50 mg, höchstens 55,00 mg, höchstens 52,50 mg, höchstens 50,00 mg, höchstens 47,50 mg, höchstens 45,00 mg oder höchstens 42,50 mg; am bevorzugtesten höchstens 40,00 mg, höchstens 27,50 mg, höchstens 35,00 mg, höchstens 32,50 mg, höchstens 30,00 mg, höchstens 27,50 mg, höchstens 25,00 mg oder höchstens 22,50 mg; und insbesondere höchstens 20,00 mg, höchstens 17,50 mg, höchstens 15,00 mg, höchstens 14,00 mg, höchstens 13,00 mg, höchstens 12,00 mg, höchstens 11,00 mg oder höchstens 10,00 mg; bevorzugt jeweils als Äquivalentdosis bezogen auf Vitamin K₁.

In einer bevorzugten Ausführungsform enthält die Darreichungsform die Vitamin-K-Komponente in einer Dosis von 0,5 mg ± 50%, bevorzugter 0,5 mg ± 40%, noch bevorzugter 0,5 mg ± 30%, am bevorzugtesten 0,5 mg ± 20%, und insbesondere 0,5 mg ± 10%, bevorzugt jeweils als Äquivalentdosis bezogen auf Vitamin K₁.

In einer bevorzugten Ausführungsform enthält die Darreichungsform die Vitamin-K-Komponente in einer Dosis von 5 mg ± 50%, bevorzugter 5 mg ± 40%, noch bevorzugter 5 mg ± 30%, am bevorzugtesten 5 mg ± 20%, und insbesondere 5 mg ± 10%, bevorzugt jeweils als Äquivalentdosis bezogen auf Vitamin K₁.

In einer anderen bevorzugten Ausführungsform enthält die Darreichungsform die Vitamin-K-Komponente in einer Dosis von 10 mg ± 50%, bevorzugter 10 mg ± 40%, noch bevorzugter 10 mg ± 30%, am bevorzugtesten 10 mg ± 20%, und insbesondere 10 mg ± 10%, bevorzugt jeweils als Äquivalentdosis bezogen auf Vitamin K₁.

In einer anderen bevorzugten Ausführungsform enthält die Darreichungsform die Vitamin-K-Komponente in einer Dosis von 15 mg ± 50%, bevorzugter 15 mg ± 40%, noch bevorzugter 15 mg ± 30%, am bevorzugtesten 15 mg ± 20%, und insbesondere 15 mg ± 10%, bevorzugt jeweils als Äquivalentdosis bezogen auf Vitamin K₁.

In einer weiteren bevorzugten Ausführungsform enthält die Darreichungsform die Vitamin-K-Komponente in einer Dosis von 20 mg ± 50%, bevorzugter 20 mg ± 40%, noch bevorzugter 20 mg ± 30%, am bevorzugtesten 20 mg ± 20%, und insbesondere 20 mg ± 10%, bevorzugt jeweils als Äquivalentdosis bezogen auf Vitamin K₁.

In einer bevorzugten Ausführungsform enthält die Darreichungsform die Nikotinamid-Komponente bevorzugt in einer Dosis von wenigstens 10 mg, wenigstens 25 mg, wenigstens 50 mg, wenigstens 75 mg, wenigstens 100 mg, wenigstens 125 mg, wenigstens 150 mg, wenigstens 175 mg oder wenigstens 200 mg; bevorzugter wenigstens 225 mg, wenigstens 250 mg, wenigstens 275 mg, wenigstens 300 mg, wenigstens 325 mg, wenigstens 350 mg, wenigstens 375 mg oder wenigstens 400 mg; noch bevorzugter wenigstens 425 mg, wenigstens 450 mg, wenigstens 475 mg, wenigstens 500 mg, wenigstens 525 mg, wenigstens 550 mg, wenigstens 575 mg oder wenigstens 600 mg; am bevorzugtesten wenigstens 625 mg, wenigstens 650 mg, wenigstens 675 mg, wenigstens 700 mg, wenigstens 725 mg, wenigstens 750 mg, wenigstens 775 mg oder wenigstens 800 mg; und insbesondere wenigstens 825 mg, wenigstens 850 mg, wenigstens 875 mg, wenigstens 900 mg, wenigstens 925 mg, wenigstens 950 mg, wenigstens 975 mg oder wenigstens 1000 mg; bevorzugt jeweils als Äquivalentdosis bezogen auf Nikotinamid.

In einer bevorzugten Ausführungsform enthält die Darreichungsform die Nikotinamid-Komponente bevorzugt in einer Dosis von höchstens 10,00 g, höchstens 9,75 g, höchstens 9,50 g, höchstens 9,25 g, höchstens 9,00 g, höchstens 8,75 g, höchstens 8,50 g oder höchstens 8,25 g; bevorzugter höchstens 8,00 g, höchstens 7,75 g, höchstens 7,50 g, höchstens 7,25 g, höchstens 7,00 g, höchstens 6,75 g, höchstens 6,50 g oder höchstens 6,25 g; noch bevorzugter höchstens 6,00 g, höchstens 5,75 g, höchstens 5,50 g, höchstens 5,25 g, höchstens 5,00 g, höchstens 4,75 g, höchstens 4,50 g oder höchstens 4,25 g; am bevorzugtesten höchstens 4,00 g, höchstens 2,75 g, höchstens 3,50 g, höchstens 3,25 g, höchstens 3,00 g, höchstens 2,75 g, höchstens 2,50 g oder höchstens 2,25 g; und insbesondere höchstens 2,00 g, höchstens 1,75 g, höchstens 1,50 g, höchstens 1,40 g, höchstens 1,30 g, höchstens 1,20 g, höchstens 1,10 g oder höchstens 1,00 g; bevorzugt jeweils als Äquivalentdosis bezogen auf Nikotinamid.

In einer bevorzugten Ausführungsform enthält die Darreichungsform die Nikotinamid-Komponente in einer Dosis von 0,01 g ± 50%, bevorzugter 0,01 g ± 40%, noch bevorzugter 0,01 g ± 30%, am bevorzugtesten 0,01 g ± 20%, und insbesondere 0,01 g ± 10%, bevorzugt jeweils als Äquivalentdosis bezogen auf Nikotinamid.

In einer anderen bevorzugten Ausführungsform enthält die Darreichungsform die Nikotinamid-Komponente in einer Dosis von 0,1 g ± 50%, bevorzugter 0,1 g ± 40%, noch bevorzugter 0,1 g ± 30%, am bevorzugtesten 0,1 g ± 20%, und insbesondere 0,1 g ± 10%, bevorzugt jeweils als Äquivalentdosis bezogen auf Nikotinamid.

In einer bevorzugten Ausführungsform enthält die Darreichungsform die Nikotinamid-Komponente in einer Dosis von 0,25 g ± 50%, bevorzugter 0,25 g ± 40%, noch bevorzugter 0,25 g ± 30%, am bevorzugtesten 0,25 g ± 20%, und insbesondere 0,25 g ± 10%, bevorzugt jeweils als Äquivalentdosis bezogen auf Nikotinamid.

In einer anderen bevorzugten Ausführungsform enthält die Darreichungsform die Nikotinamid-Komponente in einer Dosis von 0,5 g ± 50%, bevorzugter 0,5 g ± 40%, noch bevorzugter 0,5 g ± 30%, am bevorzugtesten 0,5 g ± 20%, und insbesondere 0,5 g ± 10%, bevorzugt jeweils als Äquivalentdosis bezogen auf Nikotinamid.

In einer weiteren bevorzugten Ausführungsform enthält die Darreichungsform die Nikotinamid-Komponente in einer Dosis von 1,0 g ± 50%, bevorzugter 1,0 g ± 40%, noch bevorzugter 1,0 g ± 30%, am bevorzugtesten 1,0 g ± 20%, und insbesondere 1,0 g ± 10%, bevorzugt jeweils als Äquivalentdosis bezogen auf Nikotinamid.

In einer anderen bevorzugten Ausführungsform enthält die Darreichungsform die Nikotinamid-Komponente in einer Dosis von 2,0 g ± 50%, bevorzugter 2,0 g ± 40%, noch bevorzugter 2,0 g ± 30%, am bevorzugtesten 2,0 g ± 20%, und insbesondere 2,0 g ± 10%, bevorzugt jeweils als Äquivalentdosis bezogen auf Nikotinamid.

In einer weiteren bevorzugten Ausführungsform enthält die Darreichungsform die Nikotinamid-Komponente in einer Dosis von 3,0 g ± 50%, bevorzugter 3,0 g ± 40%, noch bevorzugter 3,0 g ± 30%, am bevorzugtesten 3,0 g ± 20%, und insbesondere 3,0 g ± 10%, bevorzugt jeweils als Äquivalentdosis bezogen auf Nikotinamid.

In einer bevorzugten Ausführungsform enthält die Darreichungsform die Nikotinamid-Komponente in einer Dosis von 4,0 g ± 50%, bevorzugter 4,0 g ± 40%, noch bevorzugter 4,0 g ± 30%, am bevorzugtesten 4,0 g ± 20%, und insbesondere 4,0 g ± 10%, bevorzugt jeweils als Äquivalentdosis bezogen auf Nikotinamid.

In einer anderen bevorzugten Ausführungsform enthält die Darreichungsform die Nikotinamid-Komponente in einer Dosis von 5,0 g ± 50%, bevorzugter 5,0 g ± 40%, noch bevorzugter 5,0 g ± 30%, am bevorzugtesten 5,0 g ± 20%, und insbesondere 5,0 g ± 10%, bevorzugt jeweils als Äquivalentdosis bezogen auf Nikotinamid.

Ein Fachmann erkennt, dass es je nach Dosierung der Vitamin-K-Komponente und insbesondere der Nikotinamid-Komponente sinnvoll sein kann, die vorstehend genannte Dosis auf mehrere Dosiseinheiten aufzuteilen. Sollen beispielsweise 1,0 g Nikotinamid oral verabreicht werden, so ist dies mit Hilfe einer einzelnen Tablette nicht oder nur kaum möglich, da diese vom Patienten nicht eingenommen werden kann. In einem solchen Fall kann es von Vorteil sein, auf eine Trinklösung bzw. eine Brausetablette auszuweichen, oder aber die Gesamtdosis auf mehrere Tabletten aufzuteilen.

In einer bevorzugten Ausführungsform enthält die Darreichungsform neben der Vitamin-K-Komponente und der Nikotinamid-Komponente eine Vitamin-D-Komponente, vorzugsweise Calcitriol, bevorzugt in einer Dosis von wenigstens 0,01 µg, wenigstens 0,10 µg, wenigstens 0,25 µg, wenigstens 0,50 µg, wenigstens 0,75 µg, wenigstens 1,00 µg, wenigstens 1,25 µg, wenigstens 1,50 µg, wenigstens 1,75 µg oder wenigstens 2,00 µg; bevorzugter wenigstens 2,25 µg, wenigstens 2,50 µg, wenigstens 2,75 µg, wenigstens 3,00 µg, wenigstens 3,25 µg, wenigstens 3,50 µg, wenigstens 3,75 µg oder wenigstens 4,00 µg; noch bevorzugter wenigstens 4,25 µg, wenigstens 4,50 µg, wenigstens 4,75 µg, wenigstens 5,00 µg, wenigstens 5,25 µg, wenigstens 5,50 µg, wenigstens 5,75 µg oder wenigstens 6,00 µg; am bevorzugtesten wenigstens 6,25 µg, wenigstens 6,50 µg, wenigstens 6,75 µg, wenigstens 7,00 µg, wenigstens 7,25 µg, wenigstens 7,50 µg, wenigstens 7,75 µg oder wenigstens 8,00 µg; und insbesondere wenigstens 8,25 µg, wenigstens 8,50 µg, wenigstens 8,75 µg, wenigstens 9,00 µg, wenigstens 9,25 µg, wenigstens 9,50 µg, wenigstens 9,75 µg oder wenigstens 10,00 µg; bevorzugt jeweils als Äquivalentdosis bezogen auf Calcitriol.

In einer bevorzugten Ausführungsform enthält die Darreichungsform die Vitamin-D-Komponente, vorzugsweise Calcitriol, bevorzugt in einer Dosis von höchstens 100 µg, höchstens 97,50 µg, höchstens 95,00 µg, höchstens 92,50 µg, höchstens 90,00 µg, höchstens 87,50 µg, höchstens 85,00 µg oder höchstens 82,50 µg; bevorzugter höchstens 80,00 µg, höchstens 77,50 µg, höchstens 75,00 µg, höchstens 72,50 µg, höchstens 70,00 µg, höchstens 67,50 µg, höchstens 65,00 µg oder höchstens 62,50 µg; noch bevorzugter höchstens 60,00 µg, höchstens 57,50 µg, höchstens 55,00 µg, höchstens 52,50 µg, höchstens 50,00 µg, höchstens 47,50 µg, höchstens 45,00 µg oder höchstens 42,50 µg; am bevorzugtesten höchstens 40,00 µg, höchstens 27,50 µg, höchstens 35,00 µg, höchstens 32,50 µg, höchstens 30,00 µg, höchstens 27,50 µg, höchstens 25,00 µg oder höchstens 22,50 µg; und insbesondere höchstens 20,00 µg, höchstens 17,50 µg, höchstens 15,00 µg, höchstens 14,00 µg, höchstens 13,00 µg, höchstens 12,00 µg, höchstens 11,00 µg oder höchstens 10,00 µg; bevorzugt jeweils als Äquivalentdosis bezogen auf Calcitriol.

In einer bevorzugten Ausführungsform enthält die Darreichungsform die Vitamin-D-Komponente in einer Dosis von 0,5 µg ± 50%, bevorzugter 0,5 µg ± 40%, noch bevorzugter 0,5 µg ± 30%, am bevorzugtesten 0,5 µg ± 20%, und insbesondere 0,5 µg ± 10%, bevorzugt jeweils als Äquivalentdosis bezogen auf Calcitriol.

In einer bevorzugten Ausführungsform enthält die Darreichungsform die Vitamin-D-Komponente in einer Dosis von 1 µg ± 50%, bevorzugter 1 µg ± 40%, noch bevorzugter 1 µg ± 30%, am bevorzugtesten 1 µg ± 20%, und insbesondere 1 µg ± 10%, bevorzugt jeweils als Äquivalentdosis bezogen auf Calcitriol.

In einer bevorzugten Ausführungsform enthält die Darreichungsform die Vitamin-D-Komponente in einer Dosis von 2,5 µg ± 50%, bevorzugter 2,5 µg ± 40%, noch bevorzugter 2,5 µg ± 30%, am bevorzugtesten 2,5 µg ± 20%, und insbesondere 2,5 µg ± 10%, bevorzugt jeweils als Äquivalentdosis bezogen auf Calcitriol.

In einer bevorzugten Ausführungsform enthält die Darreichungsform die Vitamin-D-Komponente in einer Dosis von 5 µg ± 50%, bevorzugter 5 µg ± 40%, noch bevorzugter 5 µg ± 30%, am bevorzugtesten 5 µg ± 20%, und insbesondere 5 µg ± 10%, bevorzugt jeweils als Äquivalentdosis bezogen auf Calcitriol.

In einer bevorzugten Ausführungsform enthält die Darreichungsform die Vitamin-D-Komponente in einer Dosis von 7,5 µg ± 50%, bevorzugter 7,5 µg ± 40%, noch bevorzugter 7,5 µg ± 30%, am bevorzugtesten 7,5 µg ± 20%, und insbesondere 7,5 µg ± 10%, bevorzugt jeweils als Äquivalentdosis bezogen auf Calcitriol.

In einer anderen bevorzugten Ausführungsform enthält die Darreichungsform die Vitamin-D-Komponente in einer Dosis von 10 µg ± 50%, bevorzugter 10 µg ± 40%, noch bevorzugter 10 µg ± 30%, am bevorzugtesten 10 µg ± 20%, und insbesondere 10 µg ± 10%, bevorzugt jeweils als Äquivalentdosis bezogen auf Calcitriol.

Bevorzugte Ausführungsformen B¹ bis B⁴ der erfindungsgemäßen Darreichungsform, welche neben der Nikotinamid-Komponente und der Vitamin-K-Komponente zusätzlich eine Vitamin-D-Komponente umfasst, sind in nachfolgender Tabelle zusammengefasst. Dabei bedeutet N die Dosis der Nikotinamid-Komponente (bevorzugt als Äquivalentdosis bezogen auf Nikotinamid), K die Dosis der Vitamin-K-Komponente (bevorzugt als Äquivalentdosis bezogen auf Vitamin K₁) und D die Dosis der Vitamin-D-Komponente (bevorzugt als Äquivalentdosis bezogen auf Calcitriol):

| Dosis | B¹ | B² | B³ | B⁴ |
|---|---|---|---|---|
| N | 10-10.000 mg | 25-7.500 mg | 50-5.000 mg | 100-2.500 mg |
| K | 0,01-100 mg | 0,05-90 mg | 0,1-80 mg | 0,25-70 mg |
| D | 0,01-10 µg | 0,01-10 µg | 0,01-10 µg | 0,01-10 µg |

In einer bevorzugten Ausführungsform der Erfindung enthalten die erfindungsgemäße pharmazeutische Zusammensetzung und die erfindungsgemäße pharmazeutische Darreichungsform kein Vitamin A und/oder kein Vitamin B₁ und/oder kein Vitamin B₂ und/oder kein Vitamin B₆ und/oder kein Vitamin B₁₂ und/oder kein Vitamin C und/oder kein Vitamin E und/oder keine Folsäure und/oder kein Biotin und/oder keine Pantothensäure und/oder kein Selen.

Ein weiterer Aspekt der Erfindung betrifft die Verwendung von einer Vitamin-K-Komponente und einer Nikotinamid-Komponente zur Herstellung eines Medikamentes, vorzugsweise einer vorstehend beschriebenen pharmazeutischen Zusammensetzung oder einer vorstehend beschriebenen pharmazeutischen Darreichungsform, zur Vorbeugung oder Behandlung einer Erkrankung, welche mit einer extraossären, vorzugsweise kardialen oder vaskulären Kalzifizierung einhergeht, vorzugsweise zur Vorbeugung oder Verhinderung einer kardialen oder vaskulären Kalzifizierung. Bevorzugte Ausführungsformen der erfindungsgemäßen pharmazeutischen Zusammensetzung bzw. der erfindungsgemäßen pharmazeutischen Darreichungsform gelten entsprechend auch für diesen Aspekt der Erfindung.

Ein weiterer Aspekt der Erfindung betrifft die Verwendung von einer Vitamin-K-Komponente und einer Nikotinamid-Komponente zur Anwendung bei der Vorbeugung oder Behandlung einer Erkrankung, welche mit einer extraossären, vorzugsweise kardialen oder vaskulären Kalzifizierung einhergeht bzw. dadurch induziert wird, vorzugsweise zur Vorbeugung oder Verhinderung einer kardialen oder vaskulären Kalzifizierung bzw. damit assoziierten Erkrankungen. Bevorzugte Ausführungsformen der erfindungsgemäßen pharmazeutischen Zusammensetzung bzw. der erfindungsgemäßen pharmazeutischen Darreichungsform gelten entsprechend auch für diesen Aspekt der Erfindung.

Diese Aspekte der Erfindung betreffen sowohl die Prävention oder die Behandlung einer extraossären Kalzifizierung, welche im Zusammenhang mit verschiedenen Erkrankungen auftreten kann, als auch die Verhinderung von Folgeerscheinungen einer extraossären Kalzifizierung, durch Verabreichung einer Vitamin-K-Komponente und einer Nikotinamid-Komponente.

Ein weiterer Aspekt der Erfindung betrifft eine Kombination umfassend eine Vitamin-K-Komponente und eine Nikotinamid-Komponente, vorzugsweise enthalten in der erfindungsgemäßen pharmazeutischen Zusammensetzung oder der erfindungsgemäßen pharmazeutischen Darreichungsform,
- zur Verwendung bei der Vorbeugung oder Behandlung einer Erkrankung, welche mit einer extraossären Kalzifizierung einhergeht.

Bevorzugt handelt es sich bei der Kalzifizierung um Koronar-Arterien-Kalzifizierung (*Coronary Artery Calcification,* CAC).

Die Behandlung oder Vorbeugung kann auch die Verringerung einer pathologischen Kalzifizierung als solche zum Ziel haben.

Ein weiterer Aspekt der Erfindung betrifft ein Verfahren zur Vorbeugung oder Behandlung einer Erkrankung, welche mit einer extraossären, vorzugsweise kardialen oder vaskulären Kalzifizierung einhergeht bzw. dadurch induziert wird, vorzugsweise zur Vorbeugung oder Verhinderung einer kardialen oder vaskulären Kalzifizierung, welches die Verabreichung einer Vitamin-K-Komponente und einer Nikotinamid-Komponente, vorzugsweise einer vorstehend beschriebenen pharmazeutischen Zusammensetzung oder einer vorstehend beschriebenen pharmazeutischen Darreichungsform, umfasst. Bevorzugte Ausführungsformen der erfindungsgemäßen pharmazeutischen Zusammensetzung bzw. der erfindungsgemäßen pharmazeutischen Darreichungsform gelten entsprechend auch für diesen Aspekt der Erfindung.

Besonders bevorzugt eignet sich die erfindungsgemäße Zusammensetzung bzw. Darreichungsform zur Behandlung bzw. Vorbeugung von durch kardiale oder vaskuläre Kalzifizierung induzierte Erkrankungen, vorzugsweise bei Patienten mit eingeschränkter Nierenfunktion, beispielsweise Dialysepatienten, insbesondere Patienten der Hämodialyse oder Peritonealdialyse, allgemein bei allen Patienten, welche am urämischen Syndrom leiden. Diesen Patienten kann die erfindungsgemäße Zusammensetzung bzw. Darreichungsform vor, während oder nach der Hämodialyse bzw. Peritonealdialyse verabreicht werden.

In einer bevorzugten Ausführungsform wird die Vitamin-K-Komponente und/oder die Nikotinamid-Komponente der Dialyse-Zubereitung zugesetzt, so dass die erfindungsgemäße Zusammensetzung bzw. Darreichungsform in diesem Fall eine Dialyse-Zubereitung ist.

Bevorzugt ist die Erkrankung, welche mit einer extraossären Kalzifizierung einhergeht bzw. durch diese induziert wird, ausgewählt aus der Gruppe bestehend aus Arteriosklerose, Atherosklerose, koronare Herzkrankheit, chronische Herzinsuffizienz, chronische Nierenerkrankung (Grad 1 bis 5D), terminale Niereninsuffizienz, Klappenverkalkung, arterielles Aneurysma, kalzifizierende Aortenstenose, transiente cerebrale Ischämie / transiente ischämische Attacke (TIA), Schlaganfall, periphere Gefäßerkrankung, Gefäßthrombose, dentale Plaques, Zahnfleischerkrankungen (dentale Pulpasteine), Speicheldrüsensteine, Nieren- und Blasensteine, Gallensteine, Pankreas- und Darmerkrankungen (wie Pankreasgangsteine, Morbus Crohn, Colitis Ulzerosa), Lebererkrankungen (wie Leberzirrhose und Leberzysten), testikuläre Mikrosteine (Hodengries), chronische Prostatasteine, Prostatitis, Prostatakalzifizierung, extraossäre Kalzifizierung bei Hämodialysepatienten, Malakoplakie, Autoimmunerkrankungen wie Lupus Erythematodes, Sklerodermie, Dermatomyositis, Antiphospholipid Syndrom (APS), Arteriitis Nodosa, Thrombocytopenie, Hämolytische Anämie, Myelitis, Livedo reticularis, Chorea, Migräne, Juvenile Dermatomyositis, Morbus Basedow, Schilddrüsenunterfunktion, Typ I Diabetes Mellitus, Typ II Diabetes Mellitus, Addison Krankheit, Hypophysenvorderlappeninsuffizienz, plazentare und fetale Erkrankungen, polyzystische Nierenerkrankung, Glomerulopathien, Augenerkrankungen (wie corneale Kalzifizierungen, Katarakt, Makuladegeneration, retinale Gefäßerkrankungen, andere retinale Degenerationen, retinale Nervendegeneration, Retinitis, Iriitis), Ohrenerkrankungen (wie Otosklerose, Degeneration der Otolithen (Statolithen)), Erkrankungen des Vestibulaorgans und Innenohres (Vertigo und Tinnitus), thyreoglossale Zysten, Schilddrüsenzysten, ovariale Zysten, Krebs (wie Meningeom, Brustkrebs, Prostatakrebs, Schilddrüsenkrebs, seröses ovariales Adenokarcinom), Hauterkrankungen (wie dermale Kalzinosen, Kalziphylaxie, Psoriasis, Ekzeme, Lichen ruber planus), rheumatoide Arthritis, kalzifizierende Tendinitis, Osteoarthritis, Fibromyalgie, Knochensporne, diffuse interstitielle skeletale Hyperostose (DISH), intrakraniale Kalzifizierungen (wie degenerative Erkrankungen und Demenz), erythrozytäre Erkrankungen mit Anämie, Kalzifizierung der Milz, chronisch obstruktive Lungenerkrankung (COPD), Broncholithen, bronchiale Steine, Neuropathien, Kalzifizierung und Inkrustation von Implantaten, gemischt kalzifizierende Biofilme und myelodegenerative Erkrankungen (wie Multiple Sklerose, amyotrophe Lateralsklerose (ALS), Alzheimer'sche Erkrankung), jeweils bei Menschen oder Tieren.

Kardiovaskuläre Erkrankungen sind besonders bevorzugt.

Die Erfindung betrifft daher auch die Verwendung von einer Vitamin-K-Komponente und einer Nikotinamid-Komponente zur Herstellung eines Medikamentes, vorzugsweise einer vorstehend beschriebenen pharmazeutischen Zusammensetzung oder einer vorstehend beschriebenen pharmazeutischen Darreichungsform, zur Vorbeugung oder Behandlung einer Erkrankung ausgewählt aus der vorstehend definierten Markush-Gruppe.

Ein weiterer Aspekt der Erfindung betrifft ein Kit umfassend eine erste Darreichungsform, welche eine Vitamin-K-Komponente enthält, und eine zweite Darreichungsform, welche eine Nikotinamid-Komponente enthält, wobei die erste Darreichungsform und die zweite Darreichungsform vorzugsweise für unterschiedliche Arten der Verabreichung konfektioniert sind. Bevorzugt wird dir Vitamin-K-Komponente oral und die Nikotinamid-Komponente parenteral verabreicht, oder umgekehrt. Dabei können die erste und die zweite Darreichungsform simultan oder sequentiell verabreicht werden. In einer bevorzugten Ausführungsform enthält die erste Darreichungsform und/oder die zweite Darreichungsform zusätzlich eine Vitamin-D-Komponente.

Die nachfolgenden Beispiele dienen zur näheren Erläuterung der Erfindung, sind jedoch nicht einschränkend auszulegen.

### Beispiel:

Männliche Wistar-Ratten wurden *ad libitum* mit einer speziellen, adeninreichen (0,75%) und proteinarmen (2,5%) Diät gefüttert (T. Yokozawa et al., Nephron 44: 230-234 (1986); K. Tamagaki et al., Nephrol Dial Transplant (2006) 21: 651-659). Die Kalzifizierung der Femoralarterie, des renalen Gewebes und des Myokards mittels Atom-Absorptions-Spektroskopie wurde nach Kalziumextraktion der Gewebe bestimmt.

### Tiere und Haltung

Spezies: Ratte / Wistar
Stamm: Rj: WI (SPF Han)
Anbieter: Elevage Janvier, Route des Chênes Secs, 53940 Le Genest St. Isle, France
Gesundheitsstatus: SPF (*specific pathogen free*)
Bestellter Gewichtsbereich: 380 g ± 20 %
Gewöhnungsphase: wenigstens 6 Tage

### Tiergesundheit

Vor der Verabreichung der Wirkstoffe bzw. Placebos wurden die Tiere an die Laborbedingungen gewöhnt. Der Gesundheitszustand der in dieser Studie eingesetzten Tiere wurde bei Ankunft durch einen Veterinär oder eine qualifizierte Fachkraft untersucht (Eingangsuntersuchung). Es wurden nur solche Tiere an die Laborbedingungen gewöhnt, welche den entsprechenden Gesundheitsanforderungen genügten. Vor der Behandlung wurde eine weitere Gesundheitsüberprüfung durchgeführt, um sicherzustellen, dass alle Tiere nach wie vor gesund waren.

### Haltung

Unter Verwendung einer computerbasierten Methode wurden alle Tiere zufällig den jeweiligen Testgruppen zugeordnet. Die Tiere wurden individuell in Makrolon® Typ III Käfigen gehalten, welche individuell belüftet waren. Der Untersuchungsraum und die Käfige wurden in regelmäßigen Abständen gereinigt und desinfiziert. Die Raumtemperatur wurde auf 22±3 °C und die relative Luftfeuchtigkeit auf 30-70 % eingestellt. Das künstliche Licht wurde so eingestellt, dass die Tagphase 12 Stunden und die Nachtphase 12 Stunden dauerten (LD 12:12), wobei das Licht um 6:30 Uhr eingeschaltet wurde. Die Luft wurde ca. 60mal pro Stunde gewechselt und ausreichend gefiltert.

Bist zur ersten Verabreichung erhielten die Tiere *ad libitum* autoklavierte Pellets (ssniff® R/M-H; ssniff Spezialdiäten GmbH (Experimental Animal Diets Inc. 59494 Soest, Deutschland).

Mit Beginn von Tag 1 der Studie erhielten die Tiere der Gruppen 2 bis 10 eine spezielle adeninreiche (0,75%) und proteinarme (2,5%) Diät. Tiere der Gruppe 1 erhielten eine Kontrolldiät (vergleichbar mit der speziellen Diät, jedoch ohne Zusatz von Adenin und mit normalem Proteingehalt). Diese beiden Diäten wurden ebenfalls von ssniff Spezialdiäten GmbH bezogen.

Steriles und autoklaviertes Trinkwasser war jederzeit aus Trinkflaschen verfügbar *ad libitum.*

Als Einstreu für die Käfige wurde Lignocel® Typ FS14 verwendet (ssniff Spezialdiäten GmbH). Die Einstreu wurde so oft wie erforderlich erneuert und autoklaviert, um die Tiere sauber und trocken zu halten, üblicherweise einmal pro Woche.

### Experimentelles Design

Das experimentelle Design wurde behördlich geprüft und genehmigt.

### Experimentelle Gruppen und Dosierungen

Die Tiere wurden entweder täglich oder dreimal die Woche behandelt mit Substanzen bzw. Placebos gemäß nachfolgenden Tabellen 1 und 2:

**Tabelle 1:**

| Gruppe | | | Tiernummer |
|---|---|---|---|
| 1 | Kontrolle | - | 0001-0010 |
| 2 | Placebo | Placebo für Vitamin K₁ + Placebo für calcitriol + Placebo für Nikotinamid-Komponente | 0011-0020 |
| 3 | Vitamin K₁ | Vitamin K₁ Charge #1 | 0021-0030 |
| 4 | Vitamin K₁ | Vitamin K₁ Charge #2 | 0031-0040 |
| 5 | Calcitriol | Calcitriol | 0041-0050 |
| 6 | Nikotinamid-Komponente | Nikotinamid-Komponente | 0051-0060 |
| 7 | Vitamin K₁ + Calcitriol | Vitamin K₁ Charge #2 + Calcitriol | 0061-0070 |
| 8 | Vitamin K₁ + Nikotinamid-Komponente | Vitamin K₁ Charge #2 + Nikotinamid-Komponente | 0071-0080 |
| 9 | Calcitriol + Nikotinamid-Komponente | Calcitriol + Nikotinamid-Komponente | 0081-0090 |
| 10 | Vitamin K₁ + Calcitriol + Nikotinamid-Komponente | Vitamin K₁ Charge #2 + Calcitriol + Nikotinamid-Komponente | 0091-0100 |

**Tabelle 2:**

| Substanz | Dosis | Verabreichungsroute | Volumen | Häufigkeit |
|---|---|---|---|---|
| Placebo für Vitamin K₁ | - | p.o. | 3,3 ml/kg | täglich |
| Placebo für Calcitriol | - | i.p. | 3,3 ml/kg | dreimal pro Woche |
| Placebo für Nikotinamid-Komponente | - | i.p. | 3,3 ml/kg | täglich |
| Vitamin K₁ Charge #1 | 0,1 mg/kg | p.o. | 3,3 ml/kg | täglich |
| Vitamin K₁ Charge #2 | 1 mg/kg | p.o. | 3,3 ml/kg | täglich |
| Calcitriol | 20 ng/kg | i.p. | 3,3 ml/kg | dreimal pro Woche |
| Nikotinamid-Komponente | 500 mg/kg | i.p. | 3,3 ml/kg | täglich |

### Verabreichung

Das für jeden Tag zu verabreichende Gesamtvolumen wurde durch Messung des individuellen Körpergewichts dreimal die Woche ermittelt. An den Tagen, an denen keine Messung des Körpergewichts ermittelt wurde, wurde das zuletzt gemessene Körpergewicht zugrundegelegt.

Die Formulierungen von Vitamin K₁ und des Placebos für Vitamin K₁ wurden täglich p.o. mit Hilfe von Einmal-Schlundsonden über einen Gesamtzeitraum von 42 Tagen verabreicht. Calcitriol und Nikotinamid-Komponente wurden dreimal die Woche bzw. täglich i.p. verabreicht.

### Probenentnahme und Präparation von Plasma und Serum

Plasma (Li-Heparin) wurde für die klinische Chemie (Tage 15, 29 und 43 der Studie) aus Blutproben gewonnen (ca. 1,3 ml, durch retro-bulbäre Punktierung von mit Isofluoran anästhetisierten Tieren), welche sofort in Microvette Röhrchen (Sarstedt, Deutschland) überführt wurden. Alle Plasmaproben wurden bei -18 bis -30 °C gelagert, bevor die klinische Chemie gemessen wurde.

Blut zur Ermittlung von 1,25-Dihydroxy-Vitamin D₃, 25-Hydroxy-Vitamin D₃ und iPTH Konzentrationen mittels ELISA wurde durch retro-bulbäre Punktierung von mit Isofluoran anästhetisierten Tieren bei Nekropsie (Tag 43 der Studie) gewonnen. Die Blutproben wurden für 30 min bei Raumtemperatur aufbewahrt. Um Serum zu erhalten, wurden die Blutproben für 10 min bei 4 °C und 10.000 x g zentrifugiert. Aliquote der Überstände zur Ermittlung von 1,25-Dihydroxy-Vitamin D₃ Konzentrationen und 25-Hydroxy-Vitamin D₃ Konzentrationen wurden sofort bei -80 °C eingefroren und bis zur Messung aufbewahrt.

Im Detail erfolgte die Entnahme der Blutproben wie folgt:

| Gruppe | Tier-Nr. | Tage der Studie | | | |
|---|---|---|---|---|---|
| | | klinische Chemie | 1,25-Dihydroxy-Vitamin D₃ | 25-Hydroxy-Vitamin D₃ | iPTH |
| 1 | 0001-0005 | 15, 29, 43 | 43 | - | 43 |
| | 0006-0010 | | - | 43 | |
| 2 | 0011-0015 | 15, 29, 43 | 43 | - | 43 |
| | 0016-0020 | | - | 43 | |
| 3 | 0021-0025 | 15, 29, 43 | 43 | - | 43 |
| | 0026-0030 | | - | 43 | |
| 4 | 0031-0035 | 15, 29, 43 | 43 | - | 43 |
| | 0036-0040 | | - | 43 | |
| 5 | 0041-0045 | 15, 29, 43 | 43 | - | 43 |
| | 0046-0050 | | - | 43 | |
| 6 | 0051-0055 | 15, 29, 43 | 43 | - | 43 |
| | 0056-0060 | | - | 43 | |
| 7 | 0061-0065 | 15, 29, 43 | 43 | - | 43 |
| | 0066-0070 | | - | 43 | |
| 8 | 0071-0075 | 15, 29, 43 | 43 | - | 43 |
| | 0076-0080 | | - | 43 | |
| 9 | 0081-0085 | 15, 29, 43 | 43 | - | 43 |
| | 0086-0090 | | - | 43 | |
| 10 | 0091-0095 | 15, 29, 43 | 43 | - | 43 |
| | 0096-0100 | | - | 43 | |

### Probenentnahme und Präparation von Urin

Zur Urinanalyse nach vier Wochen, d.h. an Tag 29, wurden alle Tiere in metabolische Käfige gesetzt und Urinproben wurden über Nacht gesammelt (max. 16 Stunden). Während der Dauer der Sammlung des Urins fasteten die Tiere. Das Urin wurde am Tag nach seiner Sammlung analysiert.

### Nekropsie und Gewebepräparation

Am Ende des Beobachtungszeitraums (Tag 43 der Studie) wurden alle Tiere durch Induktion einer tiefen Narkose mit Isofluoran eingeschläfert. Durch retro-bulbäre Blutentnahme wurde so viel Blut wie möglich gesammelt. Nach äußerlicher Untersuchung wurde der Abdominalraum geöffnet und Veränderungen wurden registriert. Ferner wurden die folgenden Organe und Gewebe entfernt und sofort in flüssigem Stickstoff eingefroren: rechte Niere, Herz (*Myocardium*) und Femoralarterie. Die Proben wurden bei -80 °C gelagert, um schließlich den Kalziumgehalt des Gewebes mit Hilfe von Atom-Absorptions-Spektroskopie zu bestimmen.

Die Analyseergebnisse sind in Tabellen 3 bis 9 zusammengefasst:

**Tabelle 3:**

| Klinische Chemie der Plasmaproben an Tag 15 der Studie (Mittelwert ± Standardabweichung): | | | | | | | | | | | | | | | |
|---|---|---|---|---|---|---|---|---|---|---|---|---|---|---|---|
| Gruppe | ALAT [U/L] | AP [U/L] | ASAT [U/L] | CREA [µmol/L] | TP[g/L] | ALBU [g/L] | UREA [mmol/L] | CHOL [mmol/L] | TG [mmol/L] | GLUC [mmol/L] | Na [mmol/L] | K [mmol/L] | Ca [mmol/L] | Cl [mmol/L] | P [mmol/L] |
| 1 | 49,8 ± 7,3 | 310,5 ± 52,2 | 96,5 ± 17,7 | 19,9 ± 2,2 | 59,7 ± 1,0 | 30,8 ± 1,2 | 6,4 ± 0,7 | 2,7 ± 0,4 | 2,57 ± 0,85 | 8,56 ± 0,89 | 137,80 ± 1,55 | 4,93 ± 0,12 | 2,71 ± 0,10 | 100,10 ± 0,96 | 2,23 ± 0,19 |
| 2 | 43,8 ± 10,4 | 226,9 ± 116,2 | 100,7 ± 22,3 | 109,1 ± 23,5 | 52,6 ± 2,2 | 26,3 ± 1,3 | 10,0 ± 3,4 | 2,5 ± 0,3 | 1,95 ± 0,83 | 7,65 ± 1,13 | 137,00 ± 1,16 | 4,99 ± 0,50 | 2,50 ± 0,10 | 98,48 ± 2,19 | 2,18 ± 0,36 |
| 3 | 53,4 ± 6,5 | 223,6 ± 61,3 | 102,4 ± 29,7 | 113,9 ± 20,8 | 52,6 ± 2,9 | 26,8 ± 1,6 | 9,4 ± 2,4 | 2,5 ± 0,4 | 1,82 ± 0,55 | 7,57 ± 1,32 | 133,90 ± 2,08 | 4,88 ± 0,27 | 2,54 ± 0,15 | 98,21 ± 2,00 | 2,27 ± 0,26 |
| 4 | 46,6 ± 4,5 | 206,4 ± 52,2 | 89,1 ± 13,0 | 120,2 ± 26,4 | 53,9 ± 1,8 | 26,6 ± 0,8 | 8,9 ± 1,4 | 2,7 ± 0,4 | 1,14 ± 0,28 | 8,15 ± 1,87 | 133,80 ± 2,78 | 5,00 ± 0,37 | 2,59 ± 0,09 | 97,86 ± 1,40 | 2,44 ± 0,30 |
| 5 | 46,1 ± 7,1 | 160,0 ± 37,2 | 96,3 ± 34,0 | 176,3 ± 37,0 | 54,4 ± 3,6 | 26,8 ± 0,7 | 13,4 ± 4,2 | 3,0 ± 0,4 | 1,80 ± 0,80 | 6,97 ± 0,84 | 135,70 ± 1,95 | 5,20 ± 0,37 | 2,61 ± 0,14 | 95,71 ± 2,46 | 2,79 ± 0,33 |
| 6 | 61,5 ± 16,1 | 104,9 ± 13,5 | 149,2 ± 68,7 | 76,6 ± 19,6 | 53,7 ± 2,3 | 27,5 ± 1,4 | 25,4 ± 7,2 | 3,6 ± 0,5 | 2,41 ± 0,79 | 7,22 ± 0,91 | 134,30 ± 2,36 | 4,70 ± 0,44 | 2,37 ± 0,11 | 92,28 ± 2,49 | 2,24 ± 0,30 |
| 7 | 53,8 ± 11,8 | 185,3 ± 44,0 | 88,6 ± 12,7 | 110,1 ± 26,0 | 54,2 ± 3,2 | 27,6 ± 1,1 | 10,0 ± 2,8 | 2,6 ± 0,4 | 1,49 ± 0,57 | 7,18 ± 0,95 | 137,80 ± 1,32 | 4,89 ± 0,34 | 2,55 ± 0,10 | 99,14 ± 1,18 | 2,16 ± 0,26 |
| 8 | 99,3 ± 66,6 | 171,1 ± 39,2 | 142,7 ± 63,3 | 68,8 ± 26,4 | 56,0 ± 4,0 | 29,1 ± 2,1 | 16,2 ± 8,1 | 3,3 ± 0,6 | 2,35 ± 0,47 | 7,41 ± 0,58 | 135,00 ± 1,63 | 4,68 ± 0,41 | 2,57 ± 0,10 | 95,43 ± 2,70 | 2,15 ± 0,23 |
| 9 | 51,7 ± 9,7 | 113,2 ± 22,8 | 107,6 ± 19,9 | 70,9 ± 15,1 | 56,3 ± 4,1 | 28,4 ± 1,3 | 25,3 ± 5,3 | 3,4 ± 0,7 | 2,52 ± 0,87 | 7,23 ± 0,76 | 134,80 ± 1,69 | 4,64 ± 0,43 | 2,53 ± 0,11 | 92,80 ± 1,93 | 2,43 ± 0,29 |
| 10 | 79,7 ± 17,4 | 138,6 ± 39,9 | 148,3 ± 58,6 | 47,8 ± 13,3 | 56,0 ± 4,4 | 28,7 ± 2,5 | 14,1 ± 4,8 | 3,0 ± 0,6 | 1,74 ± 0,98 | 7,59 ± 0,86 | 134,29 ± 1,50 | 3,87 ± 0,33 | 2,51 ± 0,17 | 94,37 ± 2,11 | 1,97 ± 0,49 |

**Tabelle 4: - Klinische Chemie der Plasmaproben an Tag 29 der Studie (Mittelwert ± Standardabweichung):**

| Gruppe | ALAT [U/L] | AP [U/L] | ASAT [U/L] | CREA [µmol/L] | TP [g/L] | ALBU [g/L] | UREA [mmol/L] | CHOL [mmol/L] | TG [mmol/L] | GLUC [mmol/L] | Na [mmol/L] | K [mmol/L] | Ca [mmol/L] | Cl [mmol/L] | P [mmol/L] |
|---|---|---|---|---|---|---|---|---|---|---|---|---|---|---|---|
| 1 | 37,2 ± 8,5 | 138,5 ± 13,3 | 108,4 ± 28,3 | 22,2 ± 2,9 | 57,4 ± 2,1 | 30,6 ± 0,9 | 4,7 ± 0,5 | 2,5 ± 0,5 | 2,57 ± 0,85 | 9,24 ± 1,50 | 135,90 ± 0,73 | 4,60 ± 0,22 | 2,64 ± 0,07 | 101,55 ± 0,67 | 2,13 ± 0,08 |
| 2 | 30,7 ± 10,9 | 167,1 ± 36,9 | 69,2 ± 12,5 | 377,1 ± 75,0 | 55,1 ± 2,0 | 27,6 ± 1,4 | 27,8 ± 5,0 | 3,4 ± 0,5 | 1,95 ± 0,83 | 9,40 ± 1,66 | 134,33 ± 0,87 | 4,58 ± 0,19 | 2,47 ± 0,23 | 95,78 ± 1,88 | 4,70 ± 0,75 |
| 3 | 29,5 ± 6,0 | 160,9 ± 56,0 | 70,9 ± 16,3 | 457,3 ± 96,3 | 55,0 ± 2,6 | 27,2 ± 1,0 | 30,7 ± 11,3 | 3,2 ± 0,5 | 1,82 ± 0,55 | 9,62 ± 1,69 | 133,90 ± 2,08 | 4,82 ± 0,42 | 2,49 ± 0,24 | 94,76 ± 2,80 | 4,80 ± 0,73 |
| 4 | 31,5 ± 4,6 | 144,5 ± 30,8 | 69,1 ± 11,4 | 459,4 ± 99,3 | 54,8 ± 1,9 | 27,3 ± 1,3 | 24,1 ± 9,4 | 3,3 ± 0,5 | 2,02 ± 0,50 | 8,97 ± 1,58 | 133,80 ± 2,78 | 4,93 ± 0,37 | 2,41 ± 0,29 | 94,67 ± 3,25 | 4,65 ± 0,58 |
| 5 | 32,4 ± 6,9 | 174,1 ± 42,6 | 77,4 ± 13,8 | 486,5 ± 59,5 | 55,0 ± 2,0 | 27,2 ± 0,6 | 57,9 ± 18,0 | 4,0 ± 0,5 | 1,05 ± 0,48 | 8,67 ± 1,28 | 132,90 ± 2,64 | 5,32 ± 0,42 | 1,97 ± 0,30 | 90,97 ± 2,79 | 6,59 ± 0,86 |
| 6 | 43,0 ± 10,9 | 182,3 ± 69,5 | 95,1 ± 26,1 | 189,4 ± 103,6 | 54,3 ± 1,6 | 27,2 ± 0,8 | 27,8 ± 7,6 | 3,9 ± 0,4 | 1,51 ± 0,36 | 8,69 ± 1,38 | 132,90 ± 1,60 | 4,39 ± 0,38 | 2,44 ± 0,20 | 90,65 ± 2,20 | 4,18 ± 0,80 |
| 7 | 32,1 ± 5,6 | 149,9 ± 40,3 | 76,2 ± 17,4 | 394,3 ± 114,0 | 53,5 ± 1,9 | 27,1 ± 1,2 | 32,7 ± 11,5 | 3,2 ± 0,6 | 0,92 ± 0,34 | 8,55 ± 1,27 | 134,33 ± 2,55 | 4,52 ± 0,35 | 2,47 ± 0,17 | 95,46 ± 3,53 | 4,62 ± 0,81 |
| 8 | 59,1 ± 20,8 | 227,6 ± 168,2 | 139,2 ± 74,7 | 134,3 ± 33,1 | 54,6 ± 3,3 | 27,4 ± 1,7 | 18,4 ± 11,1 | 3,4 ± 0,4 | 1,86 ± 0,60 | 8,92 ± 2,02 | 133,56 ± 1,81 | 3,92 ± 0,29 | 2,55 ± 0,19 | 93,76 ± 2,72 | 2,89 ± 0,80 |
| 9 | 42,2 ± 18,1 | 164,0 ± 41,1 | 102,9 ± 21,0 | 185,5 ± 78,1 | 54,0 ± 3,3 | 26,6 ± 1,8 | 32,5 ± 8,5 | 3,7 ± 0,4 | 1,96 ± 0,98 | 7,84 ± 1,48 | 134,50 ± 2,12 | 4,64 ± 0,28 | 2,53 ± 0,21 | 92,65 ± 4,35 | 4,74 ± 1,07 |
| 10 | 57,0 ± 35,6 | 270,7 ± 176,3 | 125,3 ± 80,1 | 83,1 ± 30,8 | 55,9 ± 5,0 | 27,0 ± 2,3 | 19,0 ± ,9 | 3,5 ± 0,3 | 1,68 ± 1,78 | 8,304 ± 1,91 | 135,75 ± 4,27 | 4,34 ± 0,30 | 2,49 ± 0,14 | 95,63 ± 3,95 | 2,55 ± 0,62 |

**Tabelle 5: - Klinische Chemie der Plasmaproben an Tag 34/35 der Studie (Mittelwert ± Standardabweichung):**

| Gruppe | ALAT [U/L] | AP [U/L] | ASAT [U/L] | CREA [µmol/L] | TP [g/L] | ALBU [g/L] | UREA [mmol/L] | CHOL [mmol/L] | TG [mmol/L] | GLUC [mmol/L] | Na [mmol/L] | K [mmol/L] | Ca [mmol/L] | Cl [mmol/L] | P [mmol/L] |
|---|---|---|---|---|---|---|---|---|---|---|---|---|---|---|---|
| 9 | 51,4 ± 46,3 | 226,3 ± 69,1 | 99,9 ± 56,7 | 186,0 ± 88,7 | 54,7 ± 1,5 | 26,6 ± 0,5 | 25,5 ± 12,1 | 5,1 ± 0,7 | 3,66 ± 2,55 | 7,38 ± 1,32 | 132,75 ± 1,50 | 4,97 ± 0,28 | 2,67 ± 0,20 | 87,58 ± 2,51 | 4,49 ± 1,06 |
| 10 | 63,4 ± 34,1 | 336,7 ± 183,5 | 102,6 ± 38,1 | 126,3 ± 28,0 | 56,6 ± 6,1 | 28,7 ± 2,5 | 12,1 ± 15,9 | 3,9 ± 0,8 | 3,13 ± 1,82 | 6,89 ± 0,84 | 134,17 ± 1,94 | 4,34 ± 0,30 | 2,69 ± 0,35 | 92,03 ± 2,55 | 3,27 ± 1,05 |

**Tabelle 6:**

| Klinische Chemie der Plasmaproben an Tag 43 der Studie (Mittelwert ± Standardabweichung): | | | | | | | | | | | | | | | |
|---|---|---|---|---|---|---|---|---|---|---|---|---|---|---|---|
| Gruppe | ALAT [U/L] | AP [U/L] | ASAT [U/L] | CREA [µmol/L] | TP [g/L] | ALBU [g/L] | UREA [mmol/L] | CHOL [mmol/L] | TG [mmol/L] | GLUC [mmol/L] | Na [mmol/L] | K [mmol/L] | Ca [mmol/L] | Cl [mmol/L] | P [mmol/L] |
| 1 | 42,6 ± 7,3 | 217,1 ± 29,6 | 105,6 ± 34,1 | 20,7 ± 2,1 | 58,1 ± 2,9 | 31,2 ± 1,2 | 5,6 ± 0,6 | 2,7 ± 0,3 | 2,16 ± 0,56 | 8,74 ± 0,93 | 136,20 ± 0,92 | 5,16 ± 0,42 | 2,55 ± 0,05 | 100,55 ± 0,71 | 1,90 ± 0,10 |
| 2 | 37,4 ± 10,5 | 325,8 ± 52,1 | 73,3 ± 15,8 | 441,9 ± 65,7 | 52,4 ± 3,7 | 26,7 ± 1,2 | 42,8 ± 12,0 | 4,4 ± 0,5 | 1,48 ± 0,63 | 7,35 ± 0,51 | 133,89 ± 1,90 | 5,32 ± 0,62 | 2,29 ± 0,17 | 89,73 ± 1,64 | 4,80 ± 0,82 |
| 3 | 42,1 ± 8,9 | 385,1 ± 66,1 | 79,0 ± 18,5 | 501,9 ± 75,8 | 53,7 ± 3,2 | 27,7 ± 0,8 | 47,8 ± 21,1 | 4,3 ± 0,4 | 1,72 ± 0,63 | 7,60 ± 0,62 | 132,88 ±2,10 | 5,46 ± 0,38 | 2,23 ± 0,14 | 88,51 ± 1,91 | 4,93 ± 0,94 |
| 4 | 38,7 ± 7,9 | 298,7 ± 109,5 | 68,9 ± 14,1 | 458,6 ± 94,4 | 51,8 ± 4,1 | 26,9 ± 2,3 | 52,2 ± 18,8 | 4,3 ± 0,5 | 2,09 ± 0,82 | 7,97 ± 1,09 | 133,40 ± 1,84 | 5,31 ± 0,32 | 2,17 ± 0,27 | 89,39 ± 2,52 | 5,00 ± 0,69 |
| 5 | 41,8 ± 0,6 | 246,5 ± 7,8 | 98,8 ± 4,8 | 421,7 ± 43,6 | 51,4 ± 0,4 | 27,1 ± 1,4 | 62,7 ± 13,8 | 4,6 ± 0,4 | 3,12 ± 0,21 | 8,81 ± 0,81 | 135,50 ± 3,54 | 5,92 ± 0,16 | 2,05 ± 0,06 | 89,80 ± 5,94 | 5,56 ± 0,37 |
| 6 | n.d. | n.d. | n.d. | n.d. | n.d. | n.d. | n.d. | n.d. | n.d. | n.d. | n.d. | n.d. | n.d. | n.d. | n.d. |
| 7 | 33,2 ± 3,9 | 362,1 ± 79,6 | 91,7 ± 50,8 | 399,5 ± 58,2 | 55,4 ± 4,3 | 27,5 ± 1,2 | 44,4 ± 23,0 | 4,1 ± 0,6 | 1,95 ± 0,69 | 7,03 ± 0,77 | 134,29 ± 3,35 | 5,27 ± 0,46 | 2,35 ± 0,25 | 91,43 ± 4,3 | 5,09 ± 1,37 |
| 8 | 66,9 ± 28,5 | 226,6 ± 83,9 | 97,4 ± 29,1 | 174,7 ± 28,7 | 54,5 ± 4,2 | 27,4 ± 2,6 | 28,5 ± 7,5 | 4,7 ± 0,5 | 3,04 ± 1,38 | 7,23 ± 0,57 | 132,40 ± 3,13 | 4,49 ± 0,44 | 2,51 ± 0,23 | 90,72 ± 3,32 | 3,80 ± 0,89 |

**Tabelle 7:**

| Urinanalyse und Chemie an Tag 29 der Studie (Mittelwert ± Standardabweichung): | | | | | | |
|---|---|---|---|---|---|---|
| Gruppe | Volumen [mL] | pH | Erythrocyten [Zellen/µL] | Na [mmol/L] | Ca [mmol/L] | P [mmol/L] |
| 1 | 10,3±5,4 | 6,8±0,4 | 18±17 | 28,8±9,1 | 0,40±0,10 | 66,4±21,5 |
| 2 | 12,1±4,0 | 7,7±0,5 | 17±19 | 29,6±7,8 | 0,97±0,44 | 11,4±2,6 |
| 3 | 9,0±1,6 | 7,5±0,7 | 12±14 | 25,8±8,2 | 1,07±0,42 | 12,6±3,4 |
| 4 | 12,0±2,4 | 7,5±0,5 | 13±13 | 25,6±7,7 | 0,92±0,45 | 12,4±4,5 |
| 5 | 12,0±3,1 | 8,0±0,5 | 12±14 | 20,7±5,4 | 0,45±0,26 | 10,4±2,7 |
| 6 | 9,5±2,6 | 8,0±0,0 | 41±18 | 31,6±4,4 | 0,58±0,15 | 10,0±2,9 |
| 7 | 13,8±4,1 | 7,2±1,0 | 11±14 | 22,3±6,7 | 0,76±0,27 | 11,8±5,3 |
| 8 | 17,4±6,9 | 6,9±0,4 | 5±5 | 19,8±9,0 | 0,72±0,50 | 11,6±4,0 |
| 9 | 8,7±1,5 | 7,9±0,8 | 37±20 | 30,3±5,2 | 0,84±0,42 | 10,6±5,8 |
| 10 | 14,7±9,6 | 6,8±0,7 | 79±107 | 26,3±9,5 | 0,67±0,57 | 19,2±9,7 |

**Tabelle 8:**

| Serum 25-OH-D3, 1,25(OH)₂D3 und iPTH an Tag 43 der Studie (Mittelwert ± Standardabweichung): | | | |
|---|---|---|---|
| Gruppe | 25-OH-D3 [nmol/L] | 1,25(OH)₂D3 [pmol/L] | iPTH [µg/L] |
| 1 | 54.6±6.9 | 322.4±61.3 | 3.5±0.7 |
| 2 | 25.5±2.0 | 1.9±1.6 | 13.3±4.2 |
| 3 | 27.7±3.9 | 3.5±2.3 | 13.0±4.4 |
| 4 | 30.1±2.4 | 1.0±0.9 | 14.0±3.2 |
| 5 | 29.1±0.0 | 23.3±0.0 | 14.2±1.5 |
| 6 | n.d. | n.d. | n.d. |
| 7 | 27.1±4.5 | 12.4±11.4 | 15.1±7.7 |
| 8 | 34.7±9.2 | n.d. | 4.7±3.0 |
| 9 | 29.4±0.7 | 6.5±1.1 | 4.2±2.7 |
| 10 | 33.0±5.3 | 6.8±2.7 | 1.1±0.6 |

**Tabelle 9:**

| Kalziumkonzentrationen [µg/g feuchtes Gewebe] in Gewebe bei Nekropsie (Tag 43): | | | |
|---|---|---|---|
| Gruppe | Femoral Arterie | Niere | Herz |
| 1 | 253±200 | 97±31 | 56±23 |
| 2 | 7933±6677 | 803±530 | 193±199 |
| 3 | 7930±1446 | 859±422 | 203±137 |
| 4 | 7253±3715 | 735±416 | 202±151 |
| 5 | 12334±186 | 1675±28 | 529±281 |
| 6 | n.d. | n.d. | n.d. |
| 7 | 10006±1100 | 1828±1052 | 321±201 |
| 8 | 355±230 | 191±114 | 215±228 |
| 9 | 11944±14850 | 704±480 | 300±248 |
| 10 | 458±213 | 132±24 | 94±72 |

Wie die vorstehenden experimentellen Daten belegen, wird durch die kombinierte Verabreichung von Vitamin K₁ und die Nikotinamid-Komponente (Gruppen 8 und 10) die Kalziumkonzentration im Gewebe erheblich verringert. Die Verringerung ist wesentlich stärker, als bei Verabreichung von Vitamin K₁ alleine.

Dabei zeigt die ternäre Kombination mit Calcitriol (Gruppe 10) im Tiermodell mitunter noch bessere Ergebnisse als die binäre Kombination (Gruppe 8).

## Patentansprüche

1. Eine pharmazeutische Zusammensetzung umfassend
- eine Vitamin-K-Komponente ausgewählt aus der Gruppe bestehend aus Vitamin K₁, Vitamin K₂, Vitamin K₃, und Menadioldiphosphat; und
- eine Nikotinamid-Komponente ausgewählt aus der Gruppe bestehend aus Nikotinsäure, Nikotinamid, L-Tryptophan, NAD, NAD⁺, NADH, NADP, NADP⁺, NADPH, Acipimox, Niceritrol und Nicorandil;
wobei der Anteil der Vitamin-K-Komponente und der Nikotinamid-Komponente insgesamt wenigstens 10 Gew.-% beträgt, bezogen auf das Gesamtgewicht aller physiologisch aktiven Inhaltsstoffe der Zusammensetzung, und
wobei das relative Gewichtsverhältnis der Vitamin-K-Komponente zur Nikotinamid-Komponente im Bereich von 1: 5 bis 1: 25.000 liegt,
zur Anwendung bei der Vorbeugung oder Behandlung einer Erkrankung, welche mit einer extraossären Kalzifizierung einhergeht.

2. Die Zusammensetzung zur Anwendung nach Anspruch 1, **dadurch gekennzeichnet, dass** der Anteil der Vitamin-K-Komponente und der Nikotinamid-Komponente insgesamt wenigstens 70 Gew.-% beträgt, bezogen auf das Gesamtgewicht aller physiologisch aktiven Inhaltsstoffe der Zusammensetzung.

3. Die Zusammensetzung zur Anwendung nach einem der vorstehenden Ansprüche, **dadurch gekennzeichnet, dass** der relative Gewichtsanteil der Nikotinamid-Komponente wenigstens 1,0 Gew.-% beträgt, bezogen auf das Gesamtgewicht der Zusammensetzung.

4. Die Zusammensetzung zur Anwendung nach einem der vorstehenden Ansprüche, **dadurch gekennzeichnet, dass** sie fest, halbfest, pastös oder flüssig ist.

5. Die Zusammensetzung zur Anwendung nach einem der vorstehenden Ansprüche, **dadurch gekennzeichnet, dass** sie zusätzlich eine Vitamin-D-Komponente enthält.

6. Die Zusammensetzung zur Anwendung nach einem der vorstehenden Ansprüche, **dadurch gekennzeichnet, dass** die Erkrankung mit einer kardialen oder vaskulären Kalzifizierung einhergeht.

7. Die Zusammensetzung zur Anwendung nach einem der vorstehenden Ansprüche, **dadurch gekennzeichnet, dass** die Erkrankung, welche mit einer extraossären Kalzifizierung einhergeht, ausgewählt ist aus der Gruppe bestehend aus Arteriosklerose, Atherosklerose, koronare Herzkrankheit, chronische Herzinsuffizienz, chronische Nierenerkrankung, terminale Niereninsuffizienz, Klappenverkalkung, arterielles Aneurysma, kalzifizierende Aortenstenose, transiente cerebrale Ischämie, transiente ischämische Attacke, Schlaganfall, periphere Gefäßerkrankung, Gefäßthrombose, dentale Plaques, Zahnfleischerkrankungen, Speicheldrüsensteine, Nieren- und Blasensteine, Gallensteine, Pankreas- und Darmerkrankungen, Lebererkrankungen, testikuläre Mikrosteine, chronische Prostatasteine, Prostatitis, Prostatakalzifizierung, extraossäre Kalzifizierung bei Hämodialysepatienten, Malakoplakie, Autoimmunerkrankungen wie Lupus Erythematodes, Sklerodermie, Dermatomyositis, Antiphospholipid Syndrom, Arteriitis Nodosa, Thrombocytopenie, Hämolytische Anämie, Myelitis, Livedo reticularis, Chorea, Migräne, Juvenile Dermatomyositis, Morbus Basedow, Schilddrüsenunterfunktion, Typ I Diabetes Mellitus, Typ II Diabetes Mellitus, Addison Krankheit, Hypophysenvorderlappeninsuffizienz, plazentare und fetale Erkrankungen, polyzystische Nierenerkrankung, Glomerulopathien, Augenerkrankungen, Ohrenerkrankungen, thyreoglossale Zysten, Schilddrüsenzysten, ovariale Zysten, Krebs, Hauterkrankungen, rheumatoide Arthritis, kalzifizierende Tendinitis, Osteoarthritis, Fibromyalgie, Knochensporne, diffuse interstitielle skeletale Hyperostose, intrakraniale Kalzifizierungen, erythrozytäre Erkrankungen mit Anämie, Kalzifizierung der Milz, chronisch obstruktive Lungenerkrankung, Broncholithen, bronchiale Steine, Neuropathien, Kalzifizierung und Inkrustation von Implantaten, gemischt kalzifizierende Biofilme und myelodegenerative Erkrankungen, jeweils bei Menschen und Tieren.

8. Die Zusammensetzung zur Anwendung nach einem der vorstehenden Ansprüche, **dadurch gekennzeichnet, dass** die Erkrankung bei einem Dialysepatienten vorgebeugt oder behandelt wird.

9. Eine pharmazeutische Darreichungsform umfassend die pharmazeutische Zusammensetzung zur Anwendung nach einem der Ansprüche 1 bis 8.

10. Die Darreichungsform zur Anwendung nach Anspruch 9, **dadurch gekennzeichnet, dass** sie zur oralen oder parenteralen Verabreichung konfektioniert ist.

## Claims

1. Pharmaceutical composition comprising
- a vitamin K component selected from the group consisting of vitamin K₁, vitamin K₂, vitamin K₃ and menadiol diphosphate; and
- a nicotinamide component selected from the group consisting of nicotinic acid, nicotinamide, L-tryptophan, NAD, NAD⁺, NADH, NADP, NADP⁺, NADPH, Acipimox, Niceritrol and Nicorandil;
wherein the proportion of the vitamin K component and the nicotinamide component is in total at least 10% by weight, based on the total weight of all physiologically active ingredients of the composition, and
wherein the relative ratio by weight of the vitamin K component to the nicotinamide component is in the range from 1:5 to 1:25 000,
for use in the prevention or treatment of a disorder which accompanies extraosseous calcification.

2. Composition for use according to Claim 1, **characterized in that** the proportion of the vitamin K component and the nicotinamide component is in total at least 70% by weight, based on the total weight of all physiologically active ingredients of the composition.

3. Composition for use according to either of the preceding claims, **characterized in that** the relative proportion by weight of the nicotinamide component is at least 1.0% by weight, based on the total weight of the composition.

4. Composition for use according to any of the preceding claims, **characterized in that** said composition is solid, semi-solid, pasty or liquid.

5. Composition for use according to any of the preceding claims, **characterized in that** said composition additionally comprises a vitamin D component.

6. Composition for use according to any of the preceding claims, **characterized in that** the disease accompanies a cardiac or vascular calcification.

7. Composition for use according to any of the preceding claims, **characterized in that** the disease, which accompanies an extraosseous calcification, is selected from the group comprising arteriosclerosis, atherosclerosis, coronary heart disease, chronic heart failure, chronic kidney disease, terminal renal failure, valve calcification, arterial aneurysm, calcific aortic stenosis, transient cerebral ischaemia, transient ischaemic attacks, stroke, peripheral vascular disease, vascular thrombosis, dental plaques, gum disease, salivary gland stones, kidney and bladder stones, gall stones, pancreatic and intestinal diseases, liver diseases, testicular microlithiasis, chronic prostate stones, prostatitis, prostate calcification, extraosseous calcification in haemodialysis patients, malacoplakia, autoimmune diseases such as lupus erythematosus, scleroderma, dermatomyositis, antiphospholipid syndrome, arteritis nodosa, thrombocytopenia, haemolytic anaemia, myeltis, livedo reticularis, chorea, migraine, juvenile dermatomyositis, Morbus Basedow, hypothyroidism, type I diabetes mellitus, type II diabetes mellitus, Addison's disease, hypopituitarism, placental and foetal disorders, polycystic kidney disease, glomerulopathy, eye disorders, ear disorders, thyroglossal cysts, thyroid cysts, ovarian cysts, cancer, skin disorders, rheumatoid arthritis, calcific tendinitis, osteoarthritis, fibromyalgia, bone spurs, diffuse interstitial skeletal hyperostosis, intracranial calcification, erythrocytic disorders with anaemia, calcification of the spleen, chronic obstructive pulmonary disorder, broncholithiasis, bronchial stones, neuropathies, calcification and incrustation of implants, mixed calcific biofilms and myelodegenerative disorders, in each case in humans and animals.

8. Composition for use according to any of the preceding claims, **characterized in that** the disorder is prevented or treated in a dialysis patient.

9. Pharmaceutical dosage form comprising the pharmaceutical composition for use according to any of Claims 1 to 8.

10. Dosage form for use according to Claim 9, **characterized in that** said dosage form is formulated for oral or parenteral administration.

## Revendications

1. Composition pharmaceutique, comprenant :
- un composant vitamine K choisi dans le groupe constitué par la vitamine K₁, la vitamine K₂, la vitamine K₃ et le diphosphate de ménadiol ; et
- un composant nicotinamide choisi dans le groupe constitué par l'acide nicotinique, le nicotinamide, le L-tryptophane, le NAD, le NAD⁺, le NADH, le NADP, le NADP⁺, le NADPH, l'acipimox, le nicéritrol et le nicorandil ;
la somme des proportions du composant vitamine K et du composant nicotinamide étant au total d'au moins 10 % en poids, par rapport au poids total de tous les composants physiologiquement actifs de la composition, et
le rapport en poids relatif entre le composant vitamine K et le composant nicotinamide se situant dans la plage allant de 1:5 à 1:25 000,
destinée à une utilisation pour la prévention ou le traitement d'une maladie qui est accompagnée d'une calcification extra-osseuse.

2. Composition destinée à une utilisation selon la revendication 1, **caractérisée en ce que** la somme des proportions du composant vitamine K et du composant nicotinamide est au total d'au moins 70 % en poids, par rapport au poids total de tous les composants physiologiquement actifs de la composition.

3. Composition destinée à une utilisation selon l'une quelconque des revendications précédentes, **caractérisée en ce que** la proportion en poids relative du composant nicotinamide est d'au moins 1,0 % en poids, par rapport au poids total de la composition.

4. Composition destinée à une utilisation selon l'une quelconque des revendications précédentes, **caractérisée en ce qu'**elle est solide, semi-solide, pâteuse ou liquide.

5. Composition destinée à une utilisation selon l'une quelconque des revendications précédentes, **caractérisée en ce qu'**elle contient en outre un composant vitamine D.

6. Composition destinée à une utilisation selon l'une quelconque des revendications précédentes, **caractérisée en ce que** la maladie est accompagnée d'une calcification cardiaque ou vasculaire.

7. Composition destinée à une utilisation selon l'une quelconque des revendications précédentes, **caractérisée en ce que** la maladie qui est accompagnée d'une calcification extra-osseuse est choisie dans le groupe constitué par l'artériosclérose, l'athérosclérose, la maladie coronarienne, l'insuffisance cardiaque chronique, la maladie rénale chronique, l'insuffisance rénale terminale, la calcification de valves, l'anévrisme artériel, la sténose aortique calcifiante, l'ischémie cérébrale transitoire, les attaques ischémiques transitoires, l'accident vasculaire cérébral, la maladie vasculaire périphérique, la thrombose vasculaire, la plaque dentaire, la gingivite, les calculs des glandes salivaires, les calculs rénaux et vésicaux, les calculs biliaires, les maladies du pancréas et de l'intestin, les maladies hépatiques, les microlithiases testiculaires, les calculs prostatiques chroniques, la prostatite, la calcification de la prostate, la calcification extra-osseuse chez les patient en hémodialyse, la malakoplakie, les maladies autoimmunitaires telles que le lupus érythémateux, la sclérodermie, la dermatomyosite, le syndrome des antiphospholipides, la périartérite noueuse, la thrombocytopénie, l'anémie hémolytique, la myélyte, le livedo reticularis, la chorée, la migraine, la dermatomyosite juvénile, la maladie de Basedow, l'hypothyroïdisme, le diabète sucré de type I, le diabète sucré de type II, la maladie d'Addison, l'insuffisance antéhypophysaire, les maladies placentaires et foetales, la maladie rénale polycystique, les glomérulopathies, les maladies oculaires, les maladies auriculaires, les kystes du tractus thyréoglosse, les kystes de la thyroïde, les kystes ovariens, le cancer, les maladies de la peau, la polyarthrite rhumatoïde, la tendinite calcifiante, l'ostéoarthrite, la fibromyalgie, les ostéophytes, l'hyperostose squelettique interstitielle diffuse, les calcifications intracrâniennes, les maladies érythrocytaires avec anémie, la calcification de la rate, la maladie pulmonaire obstructive chronique, les broncholithes, les calculs bronchiaux, les neuropathies, la calcification et l'incrustation d'implants, les biofilms calcifiants mixtes et les maladies myélodégénératives, à chaque fois chez les hommes et les animaux.

8. Composition destinée à une utilisation selon l'une quelconque des revendications précédentes, **caractérisée en ce que** la maladie est prévenue ou traitée chez un patient de dialyse.

9. Forme pharmaceutique comprenant la composition pharmaceutique destinée à une utilisation selon l'une quelconque des revendications 1 à 8.

10. Forme pharmaceutique destinée à une utilisation selon la revendication 9, **caractérisée en ce qu'**elle est conditionnée pour l'administration orale ou parentérale.
